(19) Europäisches Patentamt
European Patent Office
Office européen des brevets

(11) Veröffentlichungsnummer : **0 369 212 B1**

(12) **EUROPÄISCHE PATENTSCHRIFT**

(45) Veröffentlichungstag der Patentschrift : **17.02.93 Patentblatt 93/07**

(51) Int. Cl.$^5$ : **C07C 69/736,** C07D 213/64, C07C 67/333, C07C 67/307, C07C 67/317, A01N 37/38, A01N 43/40, C07C 211/58

(21) Anmeldenummer : **89119691.7**

(22) Anmeldetag : **24.10.89**

(54) **(Hetero)Aryloxynaphthalinderivate, Verfahren und neue (Hetero)Aryloxynaphthylamine zu ihrer Herstellung sowie ihre Verwendung als Herbizide.**

(30) Priorität : **04.11.88 DE 3837464**

(43) Veröffentlichungstag der Anmeldung : **23.05.90 Patentblatt 90/21**

(45) Bekanntmachung des Hinweises auf die Patenterteilung : **17.02.93 Patentblatt 93/07**

(84) Benannte Vertragsstaaten : **BE CH DE FR GB IT LI NL**

(56) Entgegenhaltungen : **US-A- 2 547 123**

(73) Patentinhaber : **BAYER AG**
**W-5090 Leverkusen 1 Bayerwerk (DE)**

(72) Erfinder : **Andree, Roland, Dr.**
**Schillerstrasse 17**
**W-4018 Langenfeld (DE)**
Erfinder : **Haug, Michael, Dr.**
**Fahner Weg 5**
**W-5060 Bergisch-Gladbach 2 (DE)**
Erfinder : **Santel, Hans-Joachim, Dr.**
**Grünstrasse 9a**
**W-5090 Leverkusen 1 (DE)**
Erfinder : **Schmidt, Robert R., Dr.**
**Im Waldwinkel 110**
**W-5060 Bergisch-Gladbach 2 (DE)**
Erfinder : **Strang, Harry, Dr.**
**Heiderweg 53**
**W-4000 Düsseldorf 31 (DE)**

**EP 0 369 212 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue (Hetero)Aryloxynaphthalinderivate, Verfahren und neue (Hetero)Aryloxynaphthylamine zu ihrer Herstellung sowie ihre Verwendung als Herbizide.

Es ist bereits bekannt, daß bestimmte Dioxy-benzol-Derivate, wie z. B. α-(4-(2,4-Dichlor-phenoxy)-phenoxy)-propionsäure-methylester (Diclofop-nethyl) herbizid wirksam sind (vgl. DE-OS 22 23 894). Die Wirkung dieser bekannten Verbindungen gegen Unkräuter sowie ihre Kulturpflanzen-Verträglichkeit sind jedoch nicht immer zufriedenstellend.

Es wurden nun neue (Hetero)Aryloxynaphthalinderivate der allgemeinen Formel (I)

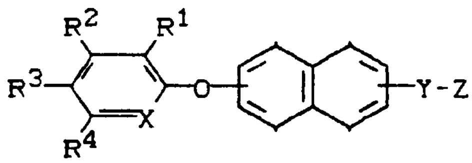
(I)

in welcher

$R^1$ für Wasserstoff, Halogen, Cyano oder Trifluormethyl steht,

$R^2$ für Wasserstoff oder Halogen steht,

$R^3$ für Halogen, Trifluormethyl, Trifluormethoxy, Trifluormethylthio oder Trifluormethylsulfonyl steht,

$R^4$ für Wasserstoff oder Halogen steht,

X für Stickstoff oder die Gruppierung C-$R^5$ steht, worin

$R^5$ für Wasserstoff oder Halogen steht,

Y für jeweils gegebenenfalls verzweigtes und/oder gegebenenfalls durch Halogen substituiertes Alkandiyl oder Alkendiyl, jeweils mit wenigstens 2 Kohlenstoffatomen, steht und

Z für Cyano oder die Gruppierung -CO-$Z^1$ steht, worin

$Z^1$ für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung -O-$R^6$ steht, worin

$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Arylthioalkyl, Arylalkoxyalkyl, Arylalkylthioalkyl, Trialkylsilylalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl, Alkylidenamino oder für ein Ammonium-, Alkylammonium-, Alkalimetall-oder Erdalkalimetall-äquivalent steht oder für die Gruppierung

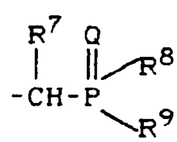

steht, worin

$R^7$ für Wasserstoff, Alkyl, Aryl, furyl, Thienyl oder Pyridyl steht,

$R^8$ für Alkyl oder Alkoxy steht,

$R^9$ für Alkoxy steht und

Q für Sauerstoff oder Schwefel steht,

oder

$R^6$ weiterhin für die Gruppierung $-(CH2)_n-R^{10}$ steht, worin

$R^{10}$ für einen gegebenenfalls durch Halogen und/oder Alkyl substituierten heterocyclischen Rest aus der Reihe furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und

n für die Zahlen 0, 1 oder 2 steht,

gefunden.

Weiter wurde gefunden, daß man die neuen (Hetero)Aryloxynaphtalinderivate der allgemeinen formel (I) erhält, wenn man

(a) (Hetero)Aryloxynaphthylamine der allgemeinen Formel (II)

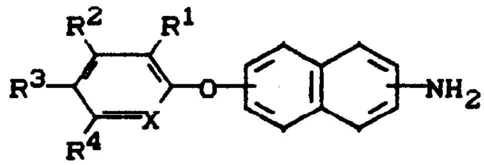

(II)

in welcher
$R^1$, $R^2$, $R^3$, $R^4$ und X die oben angegebenen Bedeutungen haben,
- oder Säureaddukte von Verbindungen der Formel (II) -
mit Natriumnitrit oder Kaliumnitrit und mit einen Hydrogenhalogenid ($HX^1$) in Gegenwart von Wasser und gegebenenfalls in Gegenwart eines organischen Lösungsmittels umsetzt und die dabei gebildeten Diazoniunsalze der allgemeinen Formel (III)

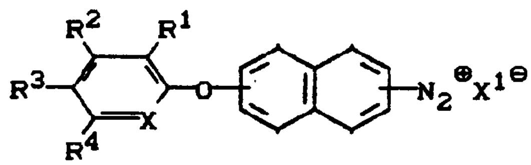

(III)

in welcher
$R^1$, $R^2$, $R^3$, $R^4$ und X die oben angegebenen Bedeutungen haben und
$X^1$ für Halogen steht,
mit Acrylsäurederivaten der allgmeinen Formel (IV)

$$Y^1 - Z^2 \quad (IV)$$

in welcher
$Y^1$ für gegebenenfalls verzweigtes und/oder gegebenenfalls durch Halogen substituiertes Alkenyl steht und
$Z^2$ für Cyano, Carboxy odër Alkoxycarbonyl steht,
in Gegenwart von Hydrogenhalogeniden ($HX^1$), gegebenenfalls in Gegenwart von Katalysatoren und gegebenenfalls in Gegenwart von Wasser und den gegebenenfalle bei der Herstellung der Verbindungen der Formel (III) verwendeten organischen Lösungsmittel umsetzt und gegebenenfalls an den so erhaltenen Verbindungen der Formel (I) weitere Derivatisierungen in den durch obige Substituentendefinition vorgegebenen Rahmen nach üblichen Methoden durchführt, oder
(b)für den Fall, daß in Formel (I) Y für gegebenenfalls verzweigtes Alkandiyl steht und $R^1$, $R^2$, $R^3$, $R^4$, X und Z die oben angegebenen Bedeutungen haben,
wenn man Verbindungen der Formel (I), in welcher Y für gegebenenfalls verzweigtes und wenigstens einfach durch Halogen substituiertes Alkandiyl oder für gegebenenfalls durch Halogen substituiertes Alkendiyl steht sowie $R^1$, $R^2$, $R^3$, $R^4$, X und Z die oben angegebenen Bedeutungen haben,
mit Wasserstoff in Gegenwart eines Hydrierungskatalysators und in Gegenwart eines Verdünnungsmittels umsetzt, oder
(c) für den Fall, daß in Formel (I) Y für durch Halogen substituiertes Alkandiyl steht und $R^1$, $R^2$, $R^3$, $R^4$, X und Z die oben angegebenen Bedeutungen haben,
wenn man Verbindungen der Formel (I), in welcher Y für Alkandiyl oder Alkendiyl steht und $R^1$, $R^2$, $R^3$, $R^4$, X und Z die oben angegebenen Bedeutungen haben,
mit Halogenierungsmitteln gegebenenfalls in Gegenwart von Katalysatoren und gegebenenfalls in Gegenwart von Verdünnungsmitteln umsetzt, oder
(d) für den Fall, daß in Formel (I) Z für die Gruppierung -CO-$Z^1$ steht, worin $Z^1$ für Hydroxy steht, und $R^1$, $R^2$, $R^3$, $R^4$, X und Y die oben angegebenen Bedeutungen haben,
wenn man Verbindungen der Formel (I), in welcher Z für Cyano oder die Gruppierung -CO-$Z^1$ steht, worin $Z^1$ für Methoxy oder Ethoxy steht, und $R^1$, $R^2$, $R^3$, $R^4$, X und Y die oben bezeichneten Bedeutungen haben,
mit Wasser, gegebenenfalls in Gegenwart eines Verseifungshilfsmittels und gegebenenfalls in Gegenwart eines organischen Lösungsmittels umsetzt, oder

(e) für den Fall, daß in Formel (I) Z für die Gruppierung -CO-$Z^1$ steht, worin $Z^1$ für Halogen steht, und $R^1$, $R^2$, $R^3$, $R^4$, X und Y die oben angegebenen Bedeutungen haben,

wenn man Verbindungen der Formel (I), in welcher Z für die Gruppierung -CO-$Z^1$ steht, worin $Z^1$ für Hydroxy steht, und $R^1$, $R^2$, $R^3$, $R^4$, X und Z die oben angegebenen Bedeutungen haben,

mit einem Halogenierungsmittel, gegebenenfalls in Gegenwart eines Katalysators und gegebenenfalls in Gegenwart eines Verdünnungsmittels, umsetzt, oder

(f) für den Fall, daß in Formel (I) Z für die Gruppierung -CO-$Z^1$ steht, worin $Z^1$ mit Ausnahme von Halogen die oben angegebene Bedeutung hat, und $R^1$, $R^2$, $R^3$, $R^4$, X und Y die oben angegebenen Bedeutungen haben,

wenn man Verbindungen der Formel (I), in welcher Z für die Gruppierung -CO-$Z^1$ steht, worin $Z^1$ für Halogen steht, und $R^1$, $R^2$, $R^3$, $R^4$, X und Y die oben angegebenen Bedeutungen haben,

mit Verbindungen der allgemeinen Formel (V)

$$H - Z^1 \quad (V)$$

in welcher

$Z^1$ mit Ausnahme von Halogen die oben angegebene Bedeutung hat,

gegebenenfalls in Gegenwart eines Säureakzeptors und gegebenenfalls in Gegenwart eines Verdünnungsmittels umsetzt, oder

(g) für den Fall, daß in Formel (I) Y für gegebenenfalls verzweigtes und/oder gegebenenfalls durch Halogen substituiertes Alkendiyl steht, und $R^1$, $R^2$, $R^3$, $R^4$, X und Z die oben angegebenen Bedeutungen haben,

wenn man Verbindungen der Formel (I), in welcher Y für wenigstens einfach durch Halogen substituiertes Alkandiyl mit wenigstens 2 Kohlenstoffatomen steht, und $R^1$, $R^2$, $R^3$, $R^4$, X und Z die oben angegebenen Bedeutungen haben,

mit Basen gegebenenfalls in Gegenwart von Verdünnungsmitteln umsetzt.

Schließlich wurde gefunden, daß die neuen (Hetero)Aryloxynaphthalinderivate der allgemeinen Formel (I) hervorragende herbizide Eigenschaften aufweisen.

Überraschenderweise sind die erfindungsgemäßen (Hetero)Aryloxynaphthalinderivate der Formel (I) gegen Unkräuter wesentlich stärker wirksam als $\alpha$-[4-(2,4-Dichlor-phenoxy)-phenoxy]-propionsäure-methylester, welches ein strukturell ähnlicher vorbekannter Wirkstoff gleicher Wirkungsrichtung ist.

Die Kohlenstoffketten in den Resten wie beispielsweise Alkyl, Alkenyl, Alkoxy, Alkylsulfonyl oder Alkoxycarbonyl sind jeweils geradkettig oder verzweigt.

Die Erfindung betrifft vorzugsweise Verbindungen der Formel (I), in welcher

$R^1$ für Wasserstoff, Fluor, Chlor, Brom, Cyano oder Trifluormethyl steht,

$R^2$ für Wasserstoff, Fluor oder Chlor steht,

$R^3$ für Fluor, Chlor, Brom, Trifluormethyl, Trifluormethoxy, Trifluormethylthio oder Trifluormethylsulfonyl steht,

$R^4$ für Wasserstoff, Fluor oder Chlor steht,

X für Stickstoff oder die Gruppierung C-$R^5$ steht, worin

$R^5$ für Wasserstoff, Fluor, Chlor oder Brom steht,

Y für jeweils gegebenenfalls verzweigtes und/oder gegebenenfalls durch Fluor, Chlor und/oder Brom substituiertes Alkandiyl oder Alkendiyl, jeweils mit 2 bis 4 Kohlenstoffatomen, steht und

Z für Cyano oder die Gruppierung -CO-$Z^1$ steht, worin

$Z^1$ für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^6$ steth, worin

$R^6$ für einen gegebenenfalls durch Fluor und/ oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Phenylaminocarbonyl-$C_1$-$C_4$-alkyl, N-($C_1$-$C_4$-Alkyl)-N-phenyl-aminocarbonyl-$C_1$-$C_4$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium-, ein Natrium-, Kalium-oder Calcium-äquivalent steht,

oder für die Gruppierung

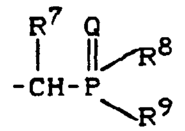

steht, worin

$R^7$ für Wasserstoff, $C_1$-$C_4$-Alkyl, Phenyl, Furyl, Thienyl oder Pyridyl steht,
$R^8$ für $C_1$-$C_4$-Alkyl oder $C_1$-$C_4$-Alkoxy steht,
$R^9$ für $C_1$-$C_4$-Alkoxy steht und
Q für Sauerstoff oder Schwefel steht,

oder

$R^6$ weiterhin für die Gruppierung $-(CH_2)_n-R^{10}$ steht, worin
n für die Zahlen 0, 1 oder 2 steht und
$R^{10}$ für einen gegebenenfalls durch fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

Die Erfindung betrifft insbesondere Verbindungen der formel (I), in welcher

$R^1$ für Cyano, Fluor oder Chlor steht,
$R^2$ für Wasserstoff, Fluor oder Chlor steht,
$R^3$ für Chlor oder Trifluormethyl steht,
$R^4$ für Wasserstoff, Fluor oder Chlor steht,
X für Stickstoff oder die Gruppierung C-$R^5$ steht, worin
$R^5$ für Wasserstoff, Fluor oder Chlor steht,
Y für jeweils gegebenenfalls durch Chlor und/oder Brom substituiertes Ethan-1,2-diyl, Propan-1,2-diyl, Ethen-1,2-diyl oder Propen-1,2-diyl steht und
Z für Cyano oder die Gruppierung -CO-$Z^1$ steht, worin
$Z^1$ für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, Diallylamino, $C_1$-$C_4$-Alkylsulfonylamino, Phenylsulfonylamino, Hydroxyamino, Cyanamino, $C_1$-$C_4$-Alkoxyamino, Hydrazino, $C_1$-$C_4$-Alkylsulfonylhydrazino, Pbenylsulfonylhydrazino, $C_1$-$C_4$-Alkylthio oder $C_1$-$C_4$-Alkoxy-carbonyl-$C_1$-$C_2$-alkylthio oder für die Gruppierung -O-$R^6$ steht, worin
$R^6$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl oder für ein Ammonium, $C_1$-$C_3$-Alkylammonium-, Natrium-, oder Kalium-äquivalent steht, oder für die Gruppierung

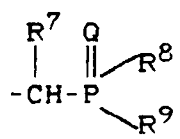

steht, worin

$R^7$ für Wasserstoff, Methyl, Phenyl, furyl, Thienyl oder Pyridyl steht,
$R^8$ für Methoxy oder Ethoxy steht,
$R^9$ für Methoxy oder Ethoxy steht und
Q für Sauerstoff oder Schwefel steht

oder

$R^6$ weiterhin für die Gruppierung $(-CH_2-)_nR^{10}$ steht, worin
n für die Zahlen 0, 1 oder 2 steht und
$R^{10}$ für einen gegebenenfalls durch Chlor und/oder Methyl substituierten heterocyclischen Rest aus der Reihe furyl, Tetrahydrofuryl, Thienyl, Perhydropyranyl, Oxazolyl, Thiazolyl und Dioxolanyl steht.

Ganz besonders bevorzugte Gruppen von Verbindungen der Formel (I) sind diejenigen der nachstehenden Formeln (IA) bis (IE), in welchen jeweils $R^1$, $R^2$, $R^3$, $R^4$, X, Y und Z die oben als insbesondere bevorzugt angegebenen Bedeutungen haben.

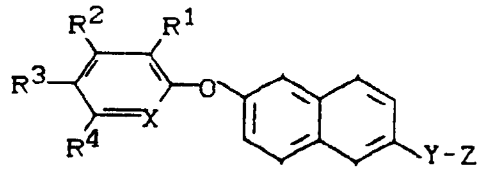

(IA)

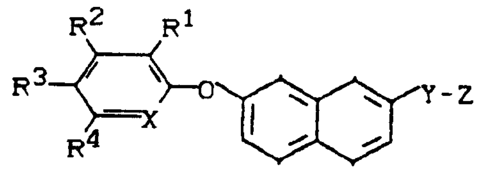

(IB)

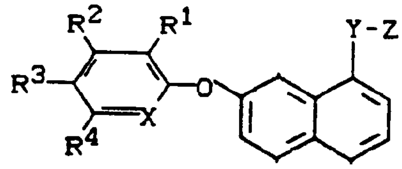

(IC)

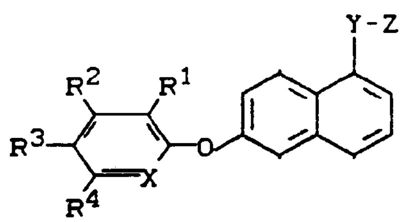

(ID)

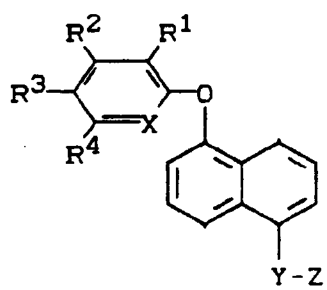

(IE)

Insbesondere bevorzugt sind diejenigen Verbindungen der Formel (I), in welchen

$R^1$ für Chlor steht,

$R^2$ für Wasserstoff steht,

$R^3$ für Trifluormethyl steht,

$R^4$ für Wasserstoff steht,

X für Stickstoff oder die Gruppierung C-$R^5$ steht, worin

$R^5$ für Fluor oder Chlor steht,

Y für jeweils gegebenenfalls durch Chlor und/oder Brom substituiertes Ethan-1,2-diyl, Propan-1,2-diyl, Ethen-1,2-diyl oder Propen-1,2-diyl steht und

Z für die Gruppierung -CO-$Z^1$ steht, worin

$Z^1$ für $C_1$-$C_4$-Alkoxy, insbesondere Methoxy,steht.

Insbesondere bevorzugt sind dabei die Gruppen von Verbindungen der Formel (IB) und (IE).

Beispielhaft sind die in der nachstehenden Tabelle 1 aufgeführten Verbindungen der Formel (I) genannt:

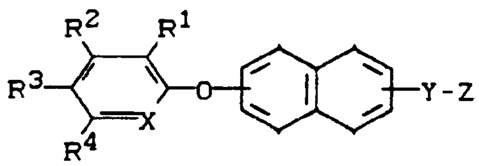

(I)

Tabelle 1

| $R^1$ | $R^2$ | $R^3$ | $R^4$ | X | Y | Z |
|---|---|---|---|---|---|---|
| Cl | H | Cl | H | CH | $-CH_2-CH-$<br>Cl | $COOCH_3$ |
| Cl | H | Cl | H | N | $-CH_2-CH-$<br>Cl | $COOC_2H_5$ |
| Cl | H | $CF_3$ | H | CH | $-CH_2-CH-$<br>Cl | $COOCH_3$ |
| Cl | H | $CF_3$ | H | CH | $-CH_2-CH-$<br>Br | $COOC_2H_5$ |
| Cl | H | $CF_3$ | H | CH | $-CH-CH-$<br>$CH_3$ Cl | $COOCH_3$ |

Tabelle 1 - Fortsetzung

| $R^1$ | $R^2$ | $R^3$ | $R^4$ | X | Y | Z |
|---|---|---|---|---|---|---|
| Cl | H | $CF_3$ | H | N | $-CH_2-CH-$<br>Cl | $COOC_2H_5$ |
| Cl | H | $CF_3$ | H | CH | $-CH=CH-$ | $COOC_2H_5$ |
| Cl | H | $CF_3$ | H | C-Cl | $-CH_2-CH-$<br>Cl | $COOCH_3$ |
| Cl | H | $CF_3$ | H | C-F | $-CH_2-CH-$<br>Cl | $COOC_2H_5$ |
| Cl | H | $CF_3$ | H | C-F | $-CH=CH-$ | $COOC_2H_5$ |
| Cl | H | $CF_3$ | H | N | $-CH=CH-$ | $COOCH_3$ |
| Cl | H | $CF_3$ | H | C-Cl | $-C=CH-$<br>$CH_3$ | $COOCH_3$ |
| Cl | H | $CF_3$ | H | C-Cl | $-CH=C-$<br>$CH_3$ | $COOCH_3$ |
| Cl | H | $CF_3$ | F | C-F | $-CH-CH_2-$<br>Br | $COOCH_3$ |
| Cl | H | $CF_3$ | H | C-F | $-C=CH-$<br>Br | COOH |
| Cl | H | $SO_2CF_3$ | H | C-Cl | $-CH=CH-$ | COOH |
| Cl | H | $SO_2CF_3$ | H | CH | $-CH_2-CH-$<br>Cl | $COOC_2H_5$ |
| Cl | H | $CF_3$ | H | CH | Cl<br>$-CH_2-C-$<br>$CH_3$ | $COOCH_3$ |

Tabelle 1 - Fortsetzung

| $R^1$ | $R^2$ | $R^3$ | $R^4$ | X | Y | Z |
|---|---|---|---|---|---|---|
| Cl | H | $CF_3$ | H | CH | 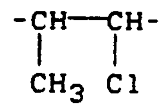 | COOH |
| Cl | H | $CF_3$ | H | C-F | 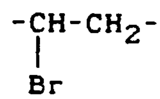 | COOH |
| Cl | H | $CF_3$ | H | C-F | 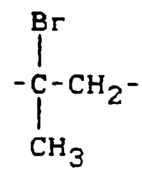 | COOH |
| Cl | H | $CF_3$ | H | C-F | 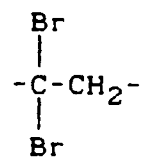 | COOH |
| Cl | H | $CF_3$ | H | C-Cl | 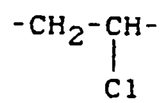 | COOH |
| Cl | H | $CF_3$ | H | N | 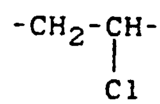 | COOH |
| Cl | H | $SO_2CF_3$ | H | C-Cl | 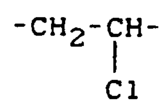 | COOH |
| Cl | H | $CF_3$ | Cl | C-Cl | -CH=CH- | COOH |
| Cl | H | Cl | H | CH | $-CH_2-CH_2-$ | $COOCH_3$ |
| Cl | H | Cl | H | N | $-CH_2-CH_2-$ | $COOC_2H_5$ |
| Cl | H | Cl | H | CH | -CH=CH- | $COOC_2H_5$ |
| Cl | H | $CF_3$ | H | CH | $-CH_2-CH_2-$ | $COOC_2H_5$ |
| Cl | H | $CF_3$ | H | N | $-CH_2-CH_2-$ | $COOC_4H_9$ |

Tabelle 1 - Fortsetzung

| $R^1$ | $R^2$ | $R^3$ | $R^4$ | X | Y | Z |
|---|---|---|---|---|---|---|
| Cl | H | $CF_3$ | H | CH | $-CH_2-CH(CH_3)-$ | $COOCH_3$ |
| Cl | H | $CF_3$ | H | CH | $-CH=CH-$ | $COOC_2H_5$ |
| Cl | H | $CF_3$ | H | CH | $-CH=C(CH_3)-$ | $COOCH_3$ |
| Cl | H | $CF_3$ | H | C-Cl | $-CH_2-CH_2-$ | $COOCH_3$ |
| Cl | H | $CF_3$ | H | C-Cl | $-CH=CH-$ | COOH |
| Cl | H | $CF_3$ | H | C-F | $-CH_2-CH_2-$ | $COOC_2H_5$ |
| Cl | H | $CF_3$ | H | C-F | $-CH=CH-$ | $COOC_2H_5$ |
| Cl | H | $CF_3$ | H | N | $-CH=CH-$ | $COOC_2H_5$ |
| Cl | H | $SO_2CF_3$ | H | CH | $-CH_2-CH_2-$ | $COOCH_3$ |
| Cl | H | $SO_2CF_3$ | H | CH | $-CH=CH-$ | COOH |
| Cl | H | $SO_2CF_3$ | H | C-Cl | $-CH_2-CH_2-$ | COOH |
| Cl | H | $SO_2CF_3$ | H | C-Cl | $-CH=CH-$ | $COOC_2H_5$ |
| Cl | H | $CF_3$ | Cl | C-Cl | $-CH_2-CH_2-$ | COOH |
| Cl | H | $CF_3$ | F | C-Cl | $-CH=CH-$ | $COOCH_3$ |
| Cl | H | $CF_3$ | F | C-F | $-CH=CH-$ | COOH |
| Cl | H | $CF_3$ | Cl | C-Cl | $-CH=CH-$ | $COOCH_3$ |
| F | H | $CF_3$ | H | C-F | $-CH_2-CH_2-$ | $COOCH_3$ |
| Cl | H | $CF_3$ | H | C-Cl | $-CH_2-CH(CH_3)-$ | COOH |
| Cl | H | $CF_3$ | H | C-Cl | $-CH=CH-$ | COCl |
| Cl | H | $CF_3$ | H | C-Cl | $-CH(CH_3)-CH_2-$ | $COCH_3$ |

Tabelle 1 - Fortsetzung

| $R^1$ | $R^2$ | $R^3$ | $R^4$ | X | Y | Z |
|---|---|---|---|---|---|---|
| Cl | H | Cl | H | CH | $-CH_2-CH_2-$ | CN |
| Cl | H | Cl | H | N | $-CH_2-CH_2-$ | CN |
| Cl | H | Cl | H | N | -CH=CH- | CN |
| Cl | H | $CF_3$ | H | CH | $-CH_2-CH_2-$ | CN |
| Cl | H | $CF_3$ | H | CH | $-CH_2-CH-$<br>\|<br>Cl | CN |
| Cl | H | $CF_3$ | H | CH | -CH=CH- | CN |
| Cl | H | $CF_3$ | H | N | $-CH_2-CH_2-$ | CN |
| Cl | H | $CF_3$ | H | C-Cl | $-CH_2-CH_2-$ | CN |
| Cl | H | $CF_3$ | H | C-Cl | $-CH_2-CH-$<br>\|<br>$CH_3$ | CN |
| Cl | H | $CF_3$ | H | C-Cl | $-CH_2-CH-$<br>\|<br>Cl | CN |
| Cl | H | $CF_3$ | H | C-Cl | -CH=CH- | CN |
| Cl | H | $CF_3$ | H | C-F | $-CH_2-CH_2-$ | CN |
| Cl | H | $CF_3$ | H | C-F | -CH=CH- | CN |
| Cl | H | $CF_3$ | H | C-F | $-CH_2-CH-$<br>\|<br>Cl | CN |
| Cl | H | $CF_3$ | H | C-Cl | $-CH-CH_2-$<br>\|<br>Br | CN |
| Cl | H | $CF_3$ | H | C-F | $-CH-CH_2-$<br>\|<br>Br | CN |

Tabelle 1 - Fortsetzung

| $R^1$ | $R^2$ | $R^3$ | $R^4$ | X | Y | Z |
|---|---|---|---|---|---|---|
| Cl | H | $CF_3$ | Cl | C-Cl | $-CH_2-CH_2-$ | CN |
| Cl | H | $CF_3$ | F | C-Cl | -CH=CH- | CN |
| Cl | H | $SO_2CF_3$ | H | CH | $-CH_2-CH-$<br>\|<br>Cl | CN |
| Cl | H | $SO_2CF_3$ | H | C-Cl | $-CH_2-CH_2-$ | CN |
| Cl | H | $CF_3$ | H | CH | -CH=C-<br>\|<br>$CH_3$ | CN |
| Cl | H | $CF_3$ | H | C-Cl | $-CH-CH_2-$<br>\|<br>$CH_3$ | CN |
| Cl | H | Cl | H | CH | $-CH_2-CH_2-$ | -CO-Cl |
| Cl | H | Cl | H | N | $-CH_2-CH_2-$ | -CO-Cl |
| Cl | H | $CF_3$ | H | CH | $-CH_2-CH_2-$ | -CO-Cl |
| Cl | H | $CF_3$ | H | CH | $-CH_2-CH-$<br>\|<br>Cl | -CO-Cl |
| Cl | H | $CF_3$ | H | CH | -CH=CH- | -CO-Cl |
| Cl | H | $CF_3$ | H | CH | $-CH_2-CH-$<br>\|<br>Br | -CO-Cl |
| Cl | H | $CF_3$ | H | CH | $-CH_2-CH-$<br>\|<br>$CH_3$ | -CO-Cl |
| Cl | H | $CF_3$ | H | CH | $-CH-CH_2-$<br>\|<br>Br | -CO-Cl |

Tabelle 1 - Fortsetzung

| $R^1$ | $R^2$ | $R^3$ | $R^4$ | X | Y | Z |
|---|---|---|---|---|---|---|
| Cl | H | $CF_3$ | H | N | $-CH_2-CH_2-$ | -CO-Cl |
| Cl | H | $CF_3$ | H | C-Cl | $-CH_2-CH_2-$ | -CO-Cl |
| Cl | H | $CF_3$ | H | C-Cl | $-CH_2-CH(Cl)-$ | -CO-Cl |
| Cl | H | $CF_3$ | H | C-Cl | -CH=CH- | -CO-Cl |
| Cl | H | $CF_3$ | H | C-F | $-CH_2-CH_2-$ | -CO-Cl |
| Cl | H | $CF_3$ | H | C-F | $-CH_2-CH(Cl)-$ | -CO-Cl |
| Cl | H | $CF_3$ | H | C-F | -CH=CH- | -CO-Cl |
| Cl | H | $CF_3$ | Cl | C-Cl | $-CH_2-CH_2-$ | -CO-Cl |
| Cl | H | $CF_3$ | F | C-Cl | -CH=CH- | -CO-Cl |
| Cl | H | $SO_2CF_3$ | H | CH | $-CH_2-CH_2-$ | -CO-Cl |
| Cl | H | $SO_2CF_3$ | H | C-Cl | $-CH_2-CH_2-$ | -CO-Cl |
| Cl | H | $CF_3$ | H | C-Cl | $-CH(CH_3)-CH_2-$ | -CO-Cl |
| Cl | H | Cl | H | CH | $-CH_2-CH(Cl)-$ | $COOCH_3$ |
| Cl | H | Cl | H | N | $-CH_2-CH(Cl)-$ | $COOC_2H_5$ |
| Cl | H | $CF_3$ | H | CN | $-CH_2-CH(Cl)-$ | CN |

Tabelle 1 - Fortsetzung

| $R^1$ | $R^2$ | $R^3$ | $R^4$ | X | Y | Z |
|---|---|---|---|---|---|---|
| Cl | H | $CF_3$ | H | CH | $-CH-CH_2-$<br>Br | $COOCH_3$ |
| Cl | H | $CF_3$ | H | N | $-CH_2-CH-$<br>Cl | $COOC_2H_5$ |
| Cl | H | $CF_3$ | H | C-Cl | $-CH_2-CH-$<br>Cl | $COOCH_3$ |
| Cl | H | $CF_3$ | H | CH | $-CH-CH-$<br>$CH_3$ Cl | CN |
| Cl | H | Cl | H | CH | $-CH_2-CH-$<br>Cl | $COOCH_3$ |
| Cl | H | Cl | H | N | $-CH_2-CH-$<br>Cl | $COOC_2H_5$ |
| Cl | H | $CF_3$ | H | CN | $-CH_2-CH-$<br>Cl | CN |
| Cl | H | $CF_3$ | H | CH | $-CH-CH_2-$<br>Br | $COOCH_3$ |
| Cl | H | $CF_3$ | H | N | $-CH_2-CH-$<br>Cl | $COOC_2H_5$ |
| Cl | H | $CF_3$ | H | C-Cl | $-CH_2-CH-$<br>Cl | $COOCH_3$ |
| Cl | H | $CF_3$ | H | CH | $-CH-CH-$<br>$CH_3$ Cl | CN |

Die in Tabelle 1 angegebenen Beispiele gelten im einzelnen für die Gruppen von Verbindungen der Formel (I), welche durch die Formeln (IA), (IB), (IC), (ID) und (IE) skizziert sind.

Verwendet man für das erfindungsgemäße Verfahren (a) 6-(2,6-Dichlor-4-trifluormethyl-phenoxy)-2-naphthylamin, Natriumnitrit und Salzsäure sowie anschließend Acrylsäure-methylester als Ausgangsstoffe, so kann der Reaktionsablauf durch das folgende Formelschema wiedergegeben werden:

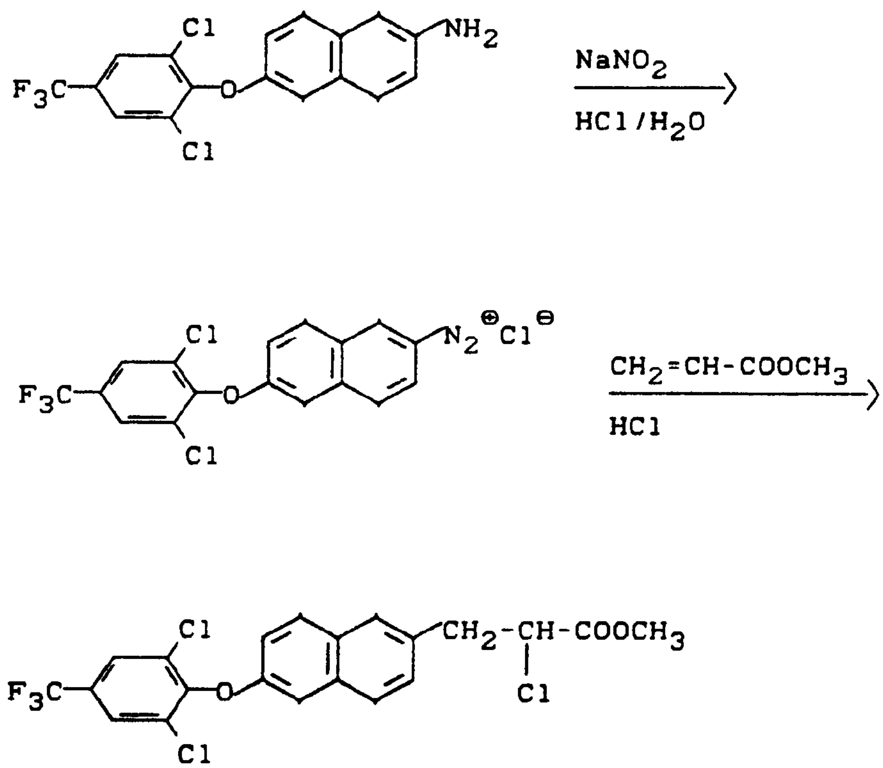


Verwendet man für das erfindungsgemäße Verfahren (b) 2-Chlor-3-[7-(3,5-Dichlor-pyridin-2-yl-oxy)-naphthalin-2-yl]-propionsäure-ethylester als Ausgangsstoff und Raney-Nickel als Katalysator, so kann der Reaktionsablauf durch das folgende Formelschema wiedergegeben werden:

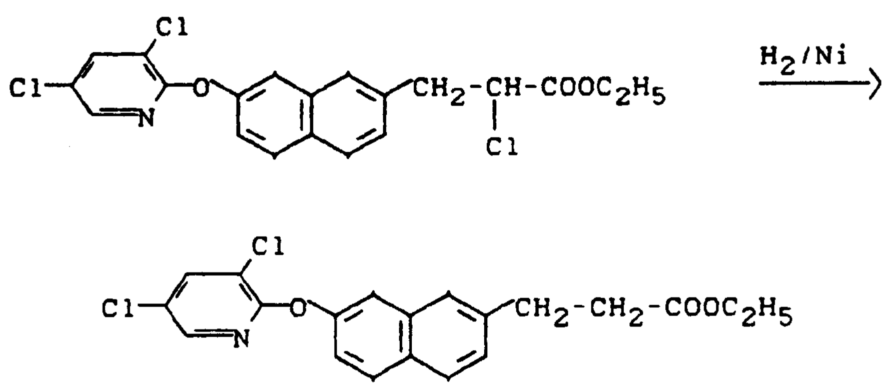


Verwendet man für das erfindungsgemäße Verfahren (c) 3-(5-(2-Fluor-4-trifluormethyl-phenoxy)-naphthalin-1-yl]-propionsäure-butylester und N-Brom-succinimid als Ausgansstoffe, so kann der Reaktionsablauf durch das folgende Formelschema wiedergegeben werden:

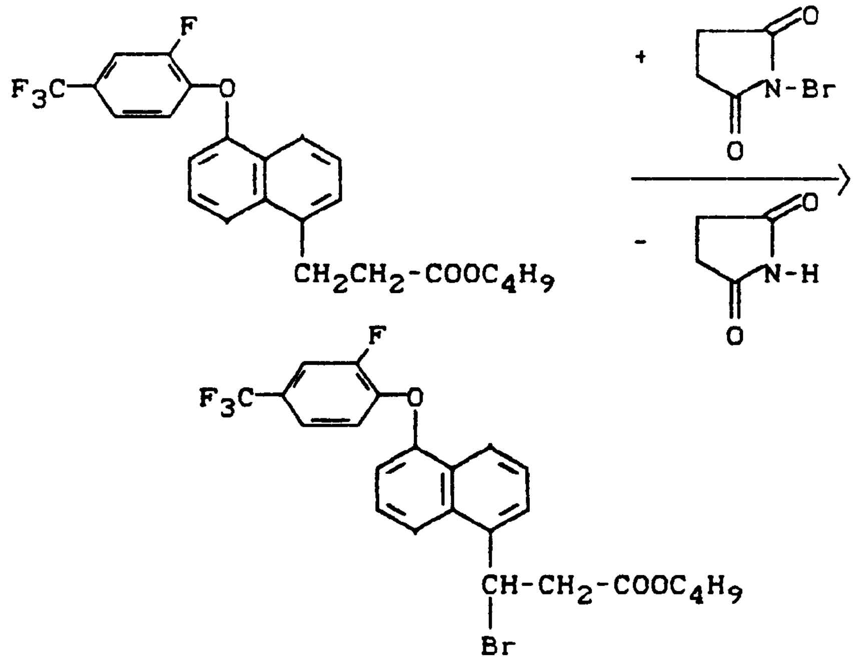

Verwendet man für das erfindungsgemäße Verfahren (d) 3-[7-(2,3,6-Trichlor-4-trifluormethyl-phenoxy)-naphthalin-1-yl]-propionsäure-nitril und Natronlauge als Ausgangsstoffe, so kann der Reaktionsablauf durch das folgende Formelschema wiedergegeben werden:

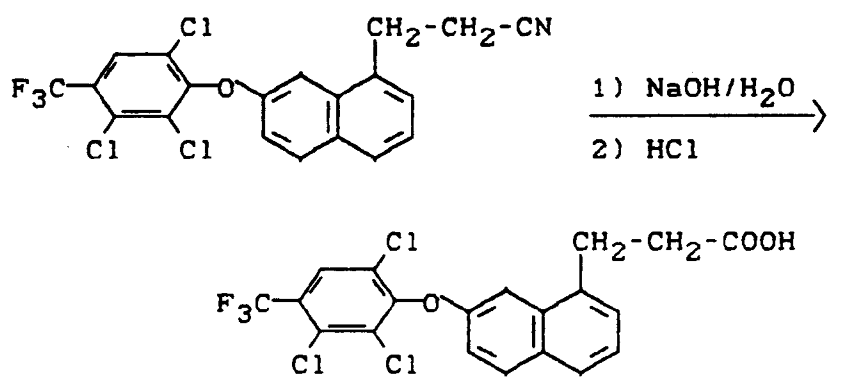


Verwendet man für das erfindungsgemäße Verfahren (e) 3-[6-(3-Chlor-5-trifluormethyl-pyridin-2-yl-oxy)-naphthalin-1-yl]-propionsäure und Thionylchlorid als Ausgangsstoffe, so kann der Reaktionsablauf durch das folgende Formelschema wiedergegeben werden:

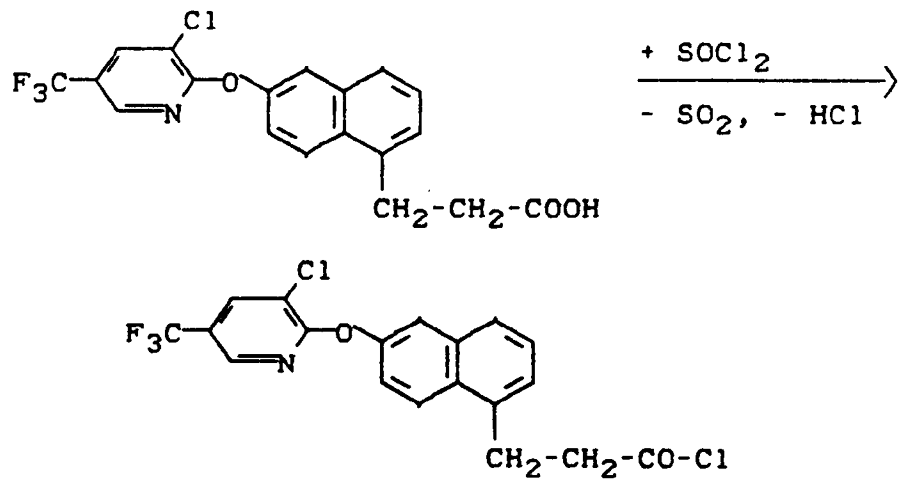


Verwendet man für das erfindungsgemäße Verfahren (f) 3-[7-(2-Chlor-6-fluor-4-trifluormethyl-phenoxy)-naphthalin-2-yl]-propionsäure-chlorid und 2-Ethoxy-ethanol als Ausgangsstoffe, so kann der Reaktionsablauf durch das folgende Formelschema wiedergegeben werden:

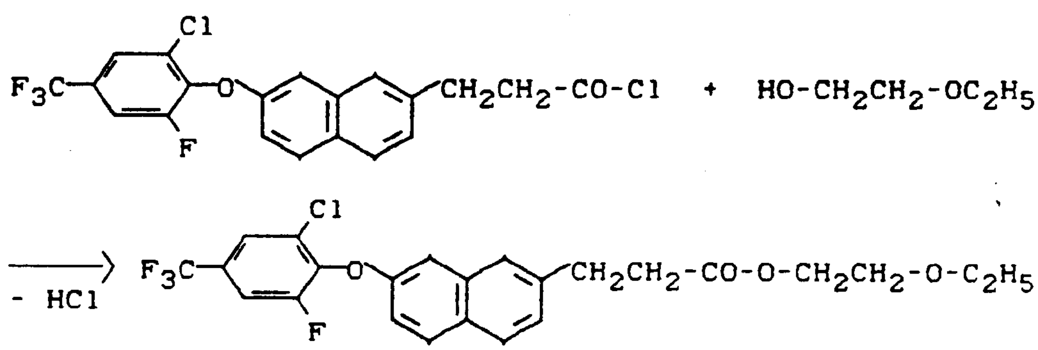


Verwendet man für das erfindungsgemäße Verfahren (g) 2-Chlor-3-[6-(2-chlor-4-trifluormethyl-phenoxy)-naphthalin-2-yl]-propionsäure-ethylester und Triethylamin als Ausgangsstoffe, so kann der Reaktionsablauf durch das folgende Formelschema wiedergegeben werden:

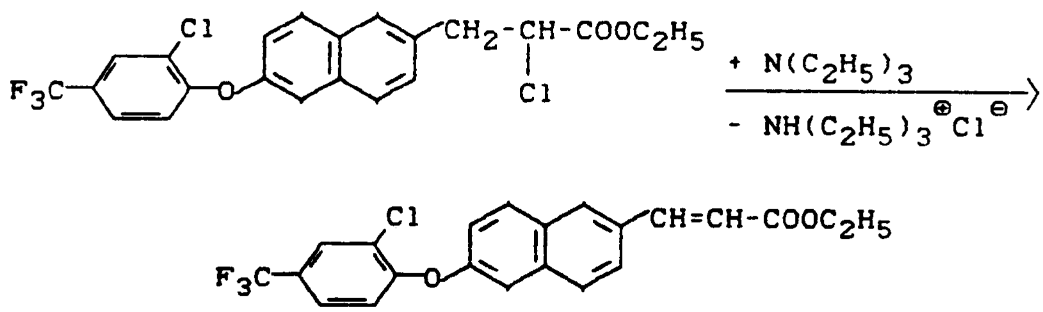


Die beim erfindungsgemäßen Verfahren (a) zur Herstellung von Verbindungen der Formel (I) als Ausgangsstoffe zu verwendenden (Hetero)Aryloxynaphthylamine sind durch die Formel (II) allgemein definiert.

In Formel (II) haben $R^1$, $R^2$, $R^3$, $R^4$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und X angegeben wurden.

Beispiele für die Ausgangsstoffe der Formel (II) sind in der nachstehenden Tabelle 2 aufgeführt.

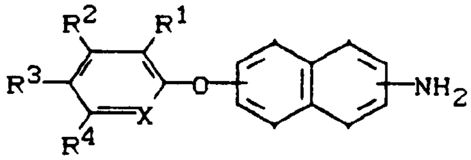

(II)

Tabelle 2: Beispiele für die Ausgangsstoffe der Formel (II)

| $R^1$ | $R^2$ | $R^3$ | $R^4$ | X |
|---|---|---|---|---|
| Cl | H | Cl | H | CH |
| Cl | H | Cl | H | N |
| Cl | H | $CF_3$ | H | CH |
| Cl | H | $CF_3$ | H | N |
| Cl | H | $CF_3$ | H | C-Cl |
| Cl | H | $CF_3$ | H | C-F |
| Cl | H | $CF_3$ | F | C-Cl |
| Cl | H | $CF_3$ | Cl | C-Cl |
| CN | H | $CF_3$ | H | CH |
| Cl | H | $SO_2CF_3$ | H | CH |
| Cl | H | $SO_2CF_3$ | H | C-Cl |
| F | H | $CF_3$ | H | C-F |

Die in Tabelle 2 angegebenen Beispiele gelten im einzelnen jeweils für alle den oben angegebenen Isomerengruppen (IA), (IB), (IC), (ID) und (IE) entsprechenden Ausgangsstoffe der Formel (II).

Ausgangsstoffe der Formel (II) sind noch nicht aus der Literatur bekannt. Man erhält die neuen (Hetero)Aryloxynaphthylamine der Formel (II), wenn man Halogen(hetero)arylverbindungen der allgemeinen Formel (VI)

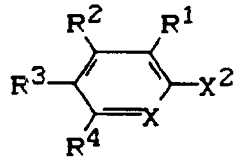

(VI)

in welcher
$R^1$, $R^2$, $R^3$, $R^4$ und X die oben angegebenen Bedeutungen haben und
$X^2$ für Fluor oder Chlor steht,
mit Hydroxynaphthylaminen der allgemeinen Formel (VII)

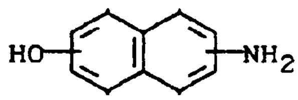

(VII)

- oder mit deren Hydrogenhalogeniden -

in Gegenwart eines Säureakzeptors, wie z. B. Natriumhydroxid oder Kaliumhydroxid, und in Gegenwart eines Verdünnungsmittels, wie z. B. Dimethylformamid, Dimethylacetamid, Dimethylsulfoxid, Tetramethylensulfon oder N-Methyl-pyrrolidon, bei Temperaturen zwischen 20°C und 150°C umsetzt und nach üblichen Methoden aufarbeitet.

Die als Ausgangsstoffe benötigten Halogen(hetero)arylverbindungen sind durch die Formel (VI) allgemein definiert. In Formel (VI) haben $R^1$, $R^2$, $R^3$, $R^4$ und X vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und X angegeben wurden.

Als Beispiele für die Halogen-(hetero)aryl-Verbindungen der Formel (VI) seien genannt:
4-Chlor-benzotrifluorid, 3,4-Dichlor-benzotrifluorid, 3,4,5-Trichlor-benzotrifluorid, 3,4-Dichlor-5-fluorbenzotrifluorid, 2,3,4,5-Tetrachlor-benzotrifluorid, 3,5-Dichlor-2,4-difluor-benzotrifluorid, 3-Chlor-4,5-difluor-benzotrifluorid sowie 2,3,5-Trichlor-pyridin und 2,3-Dichlor-5-trifluormethyl-pyridin.

Die Verbindungen der Formel (VI) sind bekannt und/oder können nach an sich bekannten Verfahren hergestellt werden (vgl. J. Chem. Soc. 1969, 211 - 217; ibid. 1971, 1547 - 1549; EP-A 34 402; US-P 4 424 396; EP-A 145 314; FR-A 2 538 380 (Chem. Abstracts 102 (1985), 61914x)).

Als Beispiele für die weiter als Ausgangsstoffe benötigten Hydroxynaphthylamine der Formel (VII) seien genannt:
5-Hydroxy-1-naphthylamin, 6-Hydroxy-1-naphthylamin, 7-Hydroxy-1-naphthylamin, 6-Hydroxy-2-naphthylamin und 7-Hydroxy-2-naphthylamin sowie deren Hydrochloride.

Die Verbindungen der Formel (VII) sind bekannte organieche Synthesechemikalien.

Die beim erfindungsgemäßen Verfahren (a) weiter als Ausgangsstoffe zu verwendenden Acrylsäurederivate sind durch die Formel (IV) allgemein definiert. In Formel (IV) stehen vorzugsweise

$Y^1$ für gegebenenfalls verzweigtes und/oder gegebenenfalls durch Fluor, Chlor und/oder Brom substituiertes Alkenyl mit 2 bis 4 Kohlenstoffatomen und

$Z^2$ für Cyano, Carboxy oder $C_1$-$C_4$-Alkoxy-carbonyl.

In Formel (IV) stehen insbesondere

$Y^1$ für gegebenenfalls durch Chlor und/oder Brom substituiertes Ethenyl oder Propenyl und

$Z^2$ für Cyano, Carboxy, Methoxycarbonyl oder Ethoxycarbonyl.

Als Beispiele für die Ausgangsstoffe der Formel (IV) seien genannt:
Acrylsäure-methylester und -ethylester, Crotonsäure-methylester und -ethylester, 2-Chlor-acrylsäure-methylester und -ethylester, 2-Brom-acrylsäure-methylester und -ethylester, Methacrylsäure-methylester und -ethylester, Acrylonitril, Methacrylonitril und 2-Chloracrylonitril.

Die Verbindungen der Formel (IV) sind bekannte organische Synthesechemikalien.

Verfahren (a) wird unter Einsatz eines Hydrogenhalogenids ($HX^1$) durchgeführt. Als Beispiele hierfür seien Hydrogenfluorid, Hydrogenchlorid, Hydrogenbromid und Hydrogeniodid genannt, Hydrogenchlorid wird vorzugsweise eingesetzt.

Verfahren (a) wird vorzugsweise unter Verwendung eines organischen Lösungsmittels durchgeführt. Insbesondere geeignet sind Ether, wie z. B. Glycoldimethylether und Diglycoldimethylether, Tetrahydrofuran und Dioxan, Ketone, wie z. B. Aceton und Methylethylketon, sowie Amide wie z. B. Dimethylformamid.

Verfahren (a) wird weiter vorzugsweise in Gegenwart von Katalysatoren duchgeführt. Als solche kommen insbesondere Kupfer und Kupferverbindungen, wie z. B. Kupfer(I)-chlorid, Kupfer(II)-chlorid, Kupfer(I)-bromid,

Kupfer(I)-iodid, Kupfer(II)-sulfat und Kupfer(II)-nitrat in Betracht.

Die Reaktionstemperaturen können bei dem erfindungsgemäßen Verfahren (a) in einem größeren Bereich variiert werden. Im allgemeinen arbeitet man bei Temperaturen zwischen -20°C und +80°C, vorzugsweise bei Temperaturen zwischen 0°C und 60°C.

Verfahren (a) wird im allgemeinen bei Normaldruck durchgeführt.

Zur Durchführung des erfindungsgemäßen Verfahrens (a) setzt man je Mol (Hetero)Aryloxynaphthylamin der Formel (II) im allgemeinen zwischen 0,8 und 2,5 Mol, vorzugsweise zwischen 1,1 und 2,0 Mol, Natriumnitrit oder Kaliumnitrit, zwischen 2 und 50 Mol, vorzugsweise zwischen 5 und 25 Mol, Hydrogenhalogenid, und zwischen 1 und 3 Mol, vorzugsweise zwischen 1,2 und 2,5 Mol, Acrylsäure-Derivat der Formel (IV) ein.

Verfahren (a) kann unter den üblichen Bedingungen der "Meerwein-Arylierung" durchgeführt werden. In einer bevorzugten Ausführungsform von Verfahren (a) wird zunächst die Ausgangsverbindung der Formel (II) in einem Verdünnungsmittel, welches zumindest Wasser und ein Hydrogenhalogenid enthält, verrührt und unter Kühlen mit einer wäßrigen Lösung von Natriumnitrit oder Kaliumnitrit diazotiert. Dann wird das Acrylsäure-Derivat der Formel (IV) und gegebenenfalls der Katalysator zum Reaktionsgemisch gegeben. Wenn - gegebenenfalls nach leichtem Erwärmen - die Stickstoff-Entwicklung abgeklungen ist, kann nach üblichen Methoden aufgearbeitet werden.

Beispielsweise wird das Reaktionsgemisch mit einem mit Wasser praktisch nicht mischbaren organischen Lösungsmittel, wie z. B. Diethylether oder Methylenchlorid,verdünnt, nach Durchschütteln die organische Phase abgetrennt, mit wäßriger Natriumhydrogencarbonat-Lösung und mit Wasser gewaschen, getrocknet und filtriert. Das nach Einengen des Filtrats als Rückstand erhaltene Rohprodukt der Formel (I) kann auf übliche Weise, beispielsweise durch Säulenchromatographie, gereinigt werden.

Die beim erfindungsgemäßen Verfahren (b) als Ausgangsstoffe zu verwendenden Verbindungen sind durch die Formel (I) mit der Maßgabe, daß Y für gegebenenfalls verzweigtes und wenigstens einfach durch Halogen substituiertes Alkandiyl oder für gegebenenfalls verzweigtes und gegebenenfalls durch Halogen substituiertes Alkendiyl steht, allgemein definiert. In diesem Fall haben $R^1$, $R^2$, $R^3$, $R^4$, X und Z vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für $R^1$, $R^2$, $R^3$, $R^4$, X und Z angegeben wurden und Y steht vorzugsweise für jeweils gegebenenfalls verzweigtes, durch Fluor, Chlor und/oder Brom substituiertes Alkandiyl oder für gegebenenfalls durch Fluor, Chlor und/oder brom substituiertes Alkendiyl, jeweils mit 2 bis 4 Kohlenstoffatomen, insbesondere für durch Chlor oder Brom substituiertes Ethan-1,2-diyl, Propan-1,2-diyl, Ethen-1,2-diyl oder Propen-1,2-diyl.

Die oben beschriebenen Ausgangsstoffe der Formel (I) für Verfahren (b) sind erfindungsgemäße, neue Verbindungen; sie können noch den erfindungsgemäßen Verfahren (a), (c), (d), (e), (f) oder (g) hergestellt werden.

Verfahren (b) wird unter Verwendung eines Hydrierungskatalysators durchgeführt. Als Beispiele für geeignete Katalysatoren seien Raney-Nickel, Platin und Palladiun genannt. Vorzugsweise wird Raney-Nickel als Katalysator für Verfahren (b) eingesetzt.

Verfahren (b) wird in Gegenwart eines Verdünnungsmittels durchgeführt. Vorzugsweise werden Kohlenwasserstoffe, wie Cyclohexan, Methylcyclohexan, Benzol, Toluol, Xylol oder Cumol, Alkohole, wie Methanol, Ethanol, Propanol oder Isopropanol, Ether, wie Glycoldimethylether, Diglycoldimethylether, Tetrahydrofuran oder Dioxan, oder Ester, wie Essigsäuremethylester oder Essigsäureethylester, oder auch Gemische der genannten Lösungsmittel als Verdünnungsmittel für Verfahren (b) eingesetzt.

Die Reaktionstemperaturen können bei dem erfindungsgemäßen Verfahren (b) in einem größeren Bereich variiert werden. Im allgemeinen arbeitet man bei Temperaturen zwischen 0°C und 150°C, vorzugsweise bei Temperaturen zwischen 20°C und 100°C.

Verfahren (b) wird im allgemeinen bei Normaldruck oder erhöhtem Druck bis etwa 200 bar, vorzugsweise bis etwa 100 bar, durchgeführt.

Verfahren (b) kann unter den bei katalytischen Hydrierungen üblichen Bedingungen durchgeführt werden. In einer bevorzugten Ausführungsform von Verfahren (b) wird die Ausgangsverbindung der Formel (I) mit dem Verdünnungsmittel und dem Katalysator vermischt und dann so lange Wasserstoff zudosiert, bis kein Verbrauch von Wasserstoff mehr festzustellen ist. Nach dem Ende der Hydrierung filtriert man das Reaktionsgemisch und erhält nach Einengen des Filtrats das Rohprodukt als Rückstand, welches auf übliche Weise, beispielsweise durch Säulenchromatographie, gereinigt werden kann.

Die beim erfindungsgemäßen Verfahren (c) als Ausgangsstoffe zu verwendenden Verbindungen sind durch die Formel (I) mit der Maßgabe, daß Y für Alkandiyl oder Alkendiyl steht, allgemein definiert. In diesem fall haben $R^1$, $R^2$, $R^3$, $R^4$, X und Z vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für $R^1$, $R^2$, $R^3$, $R^4$, X und Z angegeben wurden und Y steht vorzugsweise für jeweils

gegebenenfalls verzweigtes Alkandiyl oder Alkendiyl, jeweils mit 2 bis 4 Kohlenstoffatomen, insbesondere für Ethan-1,2-diyl, Propan-1,2-diyl, Ethen-1,2-diyl oder Propen-1,2-diyl.

Die oben beschriebenon Ausgangsstoffe der Formel (I) für Verfahren (c) sind erfindungsgemäße, neue Verbindungen; sie können nach den erfindungsgemäßen Verfahren (b), (d),(e), (f) oder (g) hergestellt werden.

Das erfindungsgemäße Verfahren (c) wird unter Verwendung von Halogenierungsmitteln durchgeführt. Es kommen die zur Halogenierung von Arylalkanen bzw. von Alkenen geeigneten Halogenierungsmittel in Betracht. Als Beispiele für bevorzugte Halogenierungsmittel seien Brom, Chlor sowie N-Brom-succinimid und N-Chlor-succinimid genannt.

Verfahren (c) wird gegebenenfalls in Gegenwart eines Katalysators durchgeführt. Als Katalysatoren kommen vorzugsweise hierbei radikalbildende Stoffe, wie z. B. 2,2′-Azoisobuttersäure-dinitril (Azoisobutyronitril), in Betracht.

Verfahren (c) wird gegebenenfalls unter Verwendung von Verdünnungenitteln durchgeführt. Als solche kommen neben Wasser vorzugsweise relativ inerte organische Solventien, wie z. B. Methylenchlorid, Chloroform, Tetrachlormethan, Chlorbenzol, o-Dichlorbenzol und Essigsäure in Betracht. Für die Umsetzung mit N-Brom-bzw. N-Chlor-succinimid wird insbesondere Tetrachlormethan als Verdünnungsmittel eingesetzt.

Die Reaktionstemperaturen können bei dem erfindungsgemäßen Verfahren (c) in einem größeren Bereich variiert werden. Im allgemeinen arbeitet man bei Temperaturen zwischen 0°C und 150°C, vorzugsweise bei Temperaturen zwischen 20°C und 100°C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in ängenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden.

Die beim erfindungsgemäßen Verfahren (d) als Ausgangsstoffe zu verwendenden Verbindungen sind durch die Formel (I) mit der Maßgabe, daß Z für Cyano oder die Gruppierung -CO-$Z^1$ steht, worin $Z^1$ für Methoxy oder Ethoxy steht, allgemein definiert. In diesem fall haben $R^1$, $R^2$, $R^3$, $R^4$, X und Y vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für $R^1$, $R^2$, $R^3$, $R^4$, X und Y angegeben wurden.

Die oben beschriebenen Ausgangsstoffe der Formel (I) für Verfahren (d) sind erfindungsgemäße, neue Verbindungen; sie können nach den erfindungsgemäßen Verfahren (a), (b), (c), (f) oder (g) hergestellt werden.

Das erfindungsgemäße Verfahren (d) wird vorzugsweise in Gegenwart eines Verseifungshilfsmittels durchgeführt. Als solche kommen insbesondere starke Säuren, wie z. B. Salzsäure oder Schwefelsäure, oder Alkalihydroxide, wie z. B. Natriumhydroxid oder Kaliumhydroxid, in Betracht.

Verfahren (d) wird in Gegenwart von Wasser und gegebenenfalls in Gegenwart eines organischen Lösungsmittels durchgeführt. Als organische Lösungsmittel werden vorzugsweise Alkohole, wie z. B, Methanol oder Ethanol, eingesetzt.

Die Reaktionstemperaturen können bei dem erfindungsgemäßen Verfahren (d) in einem größeren Bereich variiert werden. Im allgemeinen arbeitet man bei Temperaturen zwischen 10°C und 150°C, vorzugsweise bei Temperaturen zwischen 20°C und 100°C.

Das erfindungsgemäße Verfahren (d) wird im allgemeinen unter Normaldruck durchgeführt. Es ist jedoch auch möglich, unter erhöhtem oder vermindertem Druck zu arbeiten.

Zur Durchführung von Verfahren (d) werden je Mol Ausgangsverbindung der Formel (VI) im allgemeinen zwischen 0,1 und 10 Mol, vorzugsweise zwischen 0,5 und 2 Mol, Verseifungshilfsmittel eingesetzt. Die Reaktionskomponenten werden im allgemeinen bei Raumtemperatur zusammen gegeben und das Reaktionsgemisch wird, gegebenenfalls bei erhöhter Temperatur, bis zum Reaktionsende gerührt. Das gegebenenfalls nach Einengen, Abkühlen und Ansäuern kristallin anfallende Reaktionsprodukt kann durch Absaugen isoliert werden.

Die beim erfindungsgemäßen Verfahren (e) als Ausgangsstoffe zu verwendenden Verbindungen sind durch die Formel (I) mit der Maßgabe, daß Z für die Gruppierung -CO-$Z^1$ steht, worin $Z^1$ für Hydroxy steht, allgemein definiert. In diesem Fall haben $R^1$, $R^2$, $R^3$, $R^4$, X und Y vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für $R^1$, $R^2$, $R^3$, $R^4$, X und Y angegeben wurden.

Die oben beschriebenen Ausgangsstoffe der Formel (I) für Verfahren (e) sind erfindungsgemäße, neue Verbindungen; sie können nach den erfindungsgemäßen Verfahren (a), (b), (c), (d) oder (g) hergestellt werden.

Das erfindungsgemäße Verfahren (e) wird unter Verwendung eines Halogenierungsmittels durchgeführt. Es können die üblichen Mittel für die Umsetzung von Carbonsäuren zu Carbonsäurehalogeniden eingesetzt werden.

Als Beispiele hierfür seien Phosgen, Thionylchlorid, Phosphorylchlorid und Benzotrichlorid genannt. Vorzugsweise wird Thionylchlorid als Halogenierungsmittel verwendet.

Verfahren (e) wird gegebenenfalls in Gegenwart eines Katalysators durchgeführt. Es können die für die Herstellung von Säurechloriden aus Säuren üblichen Katalysatoren, wie z. B. Pyridin oder Dimethylformamid, verwendet werden.

Verfahren (e) wird gegebenenfalls in Gegenwart eines Verdünnungsmittels durchgeführt. Vorzugsweise kommen inerte organische Lösungsmittel aus der Reihe der halogenierten Kohlenwasserstoffe, wie z. B. Methylenchlorid, Chloroform, Tetrachlormethan oder 1,2-Dichlorethan, in Betracht.

Die Reaktionstemperaturen können bei dem erfindungsgemäßen Verfahren (e) in einem größeren Bereich variiert werden. Im allgemeinen arbeitet man bei Temperaturen zwischen 0°C und 100°C, vorzugsweise bei Temperaturen zwischen 10°C und 90°C.

Verfahren (e) wird im allgemeinen bei Normaldruck durchgeführt.

Zur Durchführung von Verfahren (e) werden je Mol Ausgangsverbindung der Formel (I) im allgemeinen zwischen 1 und 100 Mol, vorzugsweise zwischen 2 und 50 Mol, Halogenierungsmittel eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vernindertem Druck verbleibende Reaktionsprodukt kann durch Umkristallisation gereinigt werden, aber auch ohne weitere Reinigung für Folgeumsetzungen eingesetzt werden.

Die beim erfindungsgemäßen Verfahren (f) als Ausgangsstoffe zu verwendenden Verbindungen sind durch die Formel (I) mit der Maßgabe, daß Z für die Gruppierung -CO-$Z^1$ steht, worin $Z^1$ für Halogen steht, allgemein definiert. In diesem Fall haben $R^1$, $R^2$, $R^3$, $R^4$, X und Y vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für $R^1$, $R^2$, $R^3$, $R^4$, X und Y angegeben wurden und $Z^1$ steht vorzugsweise für Fluor, Chlor oder Brom, insbesondere für Chlor.

Die oben beschriebenen Ausgangsstoffe der Formel (I) für Verfahren (f) sind erfindungsgemäße, neue Verbindungen; sie können nach dem erfindungsgemäßen Verfahren (e) hergestellt werden.

Bei den als weitere Ausgangsstoffe für Verfahren (f) einzusetzenden Verbindungen der allgemeinen Formel (V) hat $Z^1$ vorzugsweise bzw. insbesondere diejenige Bedeutung, die bereits oben im Rahmen der Beschreibung der erfindungsgemäßen Verbindungen der Formel (I) vorzugsweise bzw. als insbesondere bevorzugt für $Z^1$ angegeben wurde, wobei Halogen ausgenommen ist.

Als Beispiele für die Verbindungen der Formel (V) seien genannt:
Methylamin, Ethylamin, Propylamin, Isopropylamin, Cyanamid, Dimethylamin, Diethylamin, Hydroxylamin, O-Methylhydroxylamin, Hydrazin, Methylsulfonylhydrazin, Methanol, Ethanol, Propanol, Isopropanol, Butanol, Isobutanol, sec-Butanol, 2-Methoxy-ethanol, 2-Ethoxy-ethanol, 2-Methylthio-ethanol, 2-Ethylthio-ethanol, 2-Benzyloxyethanol, 2-Benzylthio-ethanol, Hydroxynethanphosphonsäure-diethylester und -dimethylester, 1-Hydroxy-ethanphosphonsäure-dimethylester und -diethylester, 1-Hydroxy-1-phenyl-methanphosphonsäure-dimethylester und -diethylester, 3-Hydroxyfuran, Furfurylalkohol, Perhydrofurfurylalkohol, Milchsäure-methylester und -ethylester und Glycolsäure-methylester und -ethylester.

Diese Verbindungen sind bekannte Synthesechemikalien.

Das erfindungsgemäße Verfahren (f) wird vorzugsweise unter Verwendung von Verdünnungsmitteln durchgeführt. Als Verdünnungsmittel kommen dabei praktisch alle inerten organischen Lösungsmittel in Frage.

Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (f) alle üblicherweise für derartige Umsetzungen verwendbaren Säurebindemittel eingesetzt werden, Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (f) in einem größeren Bereich variiert werden. Im allgemeinen arbeitet man bei Temperaturen zwischen 0°C und 100°C, vorzugsweise bei Temperaturen zwischen 10° C und 50°C.

Das erfindungsgemäße Verfahren (f) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (f) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Umsetzungen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt.

Die Aufarbeitung erfolgt bei dem erfindungsgemäßen Verfahren (f) jeweils nach üblichen Methoden. Beispielsweise wird das Reaktionsgemisch - gegebenenfalls nach Einengen - mit Wasser verdünnt und das gewünschte Reaktionsprodukt wird mit einem mit Wasser praktisch nicht mischbaren organischen Lösungsmittel, z. B. Methylenchlorid, Chloroform, Diethylether, Toluol oder Xylol, extrahiert. Die organische Extraktionslösung wird mit Wasser gewaschen, mit einem üblichen Trockenmittel, wie z. B. Natriumsulfat, getrocknet und filtriert.

Nach dem Einengen des Filtrats erhält man die Verbindungen der Formel (I) als Rohprodukte, welche auf übliche Weise, z. B. chromatographisch und/oder durch Umkristallisation gereinigt werden können.

Die beim erfindungsgemäßen Verfahren (g) als Ausgangsstoffe zu verwendenden Verbindungen sind durch die Formel (I) mit der Maßgabe, daß Y für wenigstens einfach durch Halogen substituiertes Alkandiyl mit wenigstens 2 Kohlenstoffatomen steht, allgemein definiert. In diesem Fall haben $R^1$, $R^2$, $R^3$, $R^4$, X und Z vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt für $R^1$, $R^2$, $R^3$, $R^4$, X und Z angegeben wurden und Y steht vorzugsweise für gegebenenfalls verzweigtes, einfach oder zweifach durch Fluor, Chlor und/oder Brom substituiertes Alkandiyl mit 2 bis 4 Kohlenstoffatomen, insbesondere für jeweils einfach durch Chlor oder Brom substituiertes Ethan-1,2-diyl oder Propan-1,2-diyl.

Die oben beschriebenen Ausgangsstoffe der Formel (I) für Verfahren (g) sind erfindungsgemäße, neue Verbindungen; sie können nach den erfindungsgemäßen Verfahren (a), (c), (d) oder (f) hergestellt werden.

Das erfindungsgemäße Verfahren (g) wird unter Verwendung von Basen durchgsführt. Vorzugsweie werden die üblicherweise für die Eliminierung von Hydrogenhalogeniden verwendeten Basen eingesetzt.

Hierzu gehören insbesondere Alkalimetallhydroxide, wie Natriumhydroxid und Kaliumhydroxid, Alkalimetallalkoholate, wie Natriumethylat, Kaliumethylat, Natrium-tert-butylat und Kalium-tert-butylat, sowie bestimmte Amine, wie Triethylamin, Tripropylamin, Tributylamin, Ethyldiisopropylamin, Ethyldicyclohexylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Verfahren (g) wird vorzugsweise unter Verwendung von Verdünnungsmitteln durchgeführt. Als Verdünnungsmittel kommen dabei praktisch alle inerten organischen Lösungsmittel in Betracht. Hierzu gehören vorzugsweise Alkohole, wie Methanol, Ethanol, Propanol, Isopropanol, Butanol, Isobutanol, sec-Butenol und tert-Butanol sowie Etheralkohole, wie 2-Methoxy-ethanol, 2-Ethoxy-ethanol und Triethylenglycol, Ether, wie Diisopropylether, Diisobutylether, Tetrahydrofuran und Dioxan, ferner Dimethylformamid, Dimethylacetamid, N-Methylpyrrolidon, Dimethylsulfoxid und Tetramethylensulfon.

Die Reaktionstemperaturen können bei dem erfindungsgemäßen Verfahren (g) in einen größeren Bereich variiert werden. In allgemeinen arbeitet man bei Temperaturen zwischen 0°C und 250°C, vorzugsweise bei Temperaturen zwischen 50°C und 200°C.

Das erfindungsgemäße Verfahren (g) wird im allgemeinen unter Normaldruck durchgeführt. Eu ist jedoch auch möglich, unter erhöhtem oder vermindertem Druck zu arbeiten.

Zur Durchführung des erfindungsgemäßen Verfahrens (g) setzt man je Mol Ausgangsverbindung der Formel (I) im allgemeinen zwischen 1 und 3 Mol, vorzugsweise zwischen 1,2 und 2,5 Mol, einer Base ein. Die Reaktionskomponenten werden im allgemeinen bei Raumtemperatur zusammengegeben und das Reaktionsgemisch wird dann im allgemeinen bei erhöhter Temperatur bis zum Ende der Umsetzung gerührt. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium,

Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen, vor allem im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bins, Sepiolith, Dolonit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in frage: z.B. nichtionogene und anionische Emulgatoren, wie Polyoxyethylen-fettsäure-Ester, Polyoxyethylen-fettalkohol-Ether, z.B. Alkylaryl-polyglykolether, Alkylsulfonate, Alkylsulfate, Arylsulfonate sowie Eiweißhydrolysate; als Dispergiermittel kommen in frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide, Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur

Unkrautbekämpfung in Zuckerrüben und 4-Amino-6-(1,1-dimethyl-ethyl)-3-methylthio-1,2,4-triazin-5(4H)-on (METRIBUZIN) zur Unkrautbekämpfung in Sojabohnen, in frage; 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)harnstoff (CHLORTOLURON); 2-[4-(2,4-Dichlorphenoxy)phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE) und 3-[[[[(4-Methoxy-6-methyl-1,3,5-triazin-2-yl)-amino]-carbonyl]-amino]-sulfonyl]-thiophen-2-carbonsäure-methylester (THIAMETURON). Einige Mischungen zeigen überreschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden.

Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffnenge kann in einen größeren Bereich schwanken. Sie hängt in wesentlichen von der Art des gewünschten Effektes ab. In allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

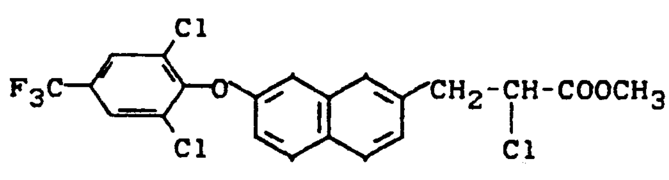

(Verfahren (a))

14,6 g (0,039 Mol) 7-(2,6-Dichlor-4-trifluormethyl-phenoxy)-2-aminonaphthalin werden in 80 ml Aceton und 50 ml konz. Salzsäure gelöst. Unter Kühlen auf 0 - 5°C werden innerhalb von 30 Minuten 3 g (0,043 Mol) Natriumnitrit, gelöst in 4 ml Wasser, zugetropft. Nach 5minütigem Nachrühren werden weiterhin unter Kühlung zunächst 4,7 g (0,054 Mol) Acrylsäuremethylester, nach weiteren 40 Minuten 0,6 g Kupfer(II)chlorid zugefügt. Man läßt das Reaktionsgemisch über Nacht auf Raumtemperatur kommen und gießt es dann in Methylenchlorid/Wasser ein. Die organische Phase wird eingedampft und der Rückstand über Kieselgel mit Toluol/Hexan (1 : 1) chromatographiert.

Man erhält 2,2 g (12 % der Theorie) 2-Chlor-3-[7-(2,6-dichlor-4-trifluormethyl-phenoxy)-naphthalin-2-yl]-propionsäure-methylester in Form orangefarbener Kristalle vom Schmelzpunkt 136°C.

Beispiel 2

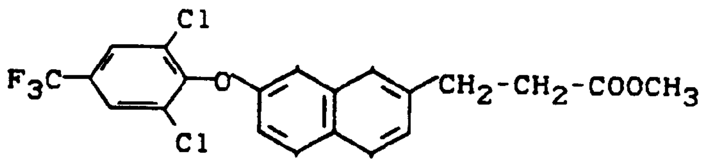

(Verfahren (b))

1,3 g (0,0025 Mol) 2-Chlor-3-[7-(2,6-dichlor-4-trifluormethyl-phenoxy)-naphthalin-2-yl]-propionsäuremethylester werden in einer Mischung aus 50 ml Methanol und 50 ml Toluol aufgenommen und nach Zugabe von 1,0 g Raney-Nickel 2 Stunden bei 40°C und 50 bar hydriert. Anschließend wird filtriert und vom Filtrat wird das Lösungsmittel im Wasserstrahlvakuum sorgfältig abdestilliert.

Man erhält 0,5 g (45 %, der Theorie) 3-[7-(2,6-Dichlor-4-trifluormethyl-phenoxy)-naphthalin-2-yl]-propionsäuremethylester als öligen Rückstand.

$^1$H-NMR ($CDCl_3$,δ): 2,65 (t) und 3,05 (t) (-$C\underline{H}_2$-$C\underline{H}_2$-$COOCH_3$)

Analog zu den Beispielen 1 und 2 sowie nach der allgemeinen Beschreibung der erfindungsgemäßen Herstellungsverfahren können die in der nachstehenden Tabelle 3 aufgeführten Verbindungen der Formel (I) erhalten werden, welche genauer durch die "Isomerengruppen" der Formeln (IA), (IB), (IC), (ID) und (IE) spezifiziert sind.

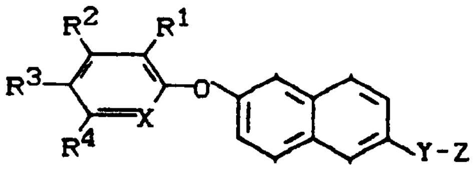

(IA)

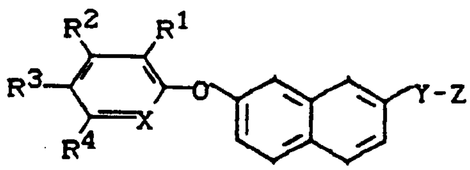

(IB)

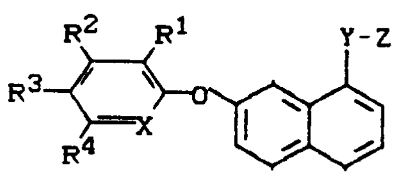

(IC)

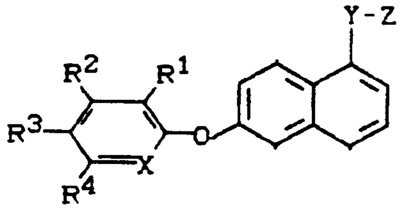

(ID)

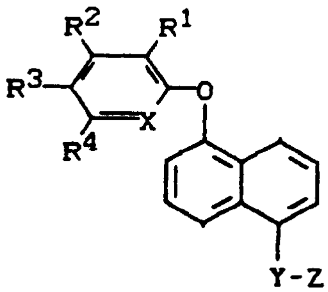

(IE)

<u>Tabelle 3:</u> Beispiele für Verbindungen der Formel (I)

| Beisp.-Nr. | Isomeren-gruppe | $R^1$ | $R^2$ | $R^3$ | $R^4$ | X | Y | Z | Physikalische Daten |
|---|---|---|---|---|---|---|---|---|---|
| 3 | IB | Cl | H | $CF_3$ | H | C-F | $-CH_2-CH-$<br>\|<br>Cl | $COOCH_3$ | δ:4,53(t)(-CH-)<br>\|<br>Cl |
| 4 | IB | Cl | H | $CF_3$ | H | C-F | $-CH_2-CH_2-$ | $COOCH_3$ | δ:2,69 und 3,08 $(-CH_2-CH_2-)$ |
| 5 | IB | Cl | H | $CF_3$ | H | C-Cl | -CH—CH-<br>\| \|<br>$CH_3$ Cl | $COOCH_3$ | |
| 6 | IB | Cl | H | $CF_3$ | H | C-Cl | $-CH-CH_2-$<br>\|<br>$CH_3$ | $COOCH_3$ | |
| 7 | IB | Cl | H | $CF_3$ | H | C-Cl | $CH_3$<br>\|<br>$-CH_2-C-$<br>\|<br>Cl | $COOCH_3$ | Fp. 133 °C |
| 8 | IB | Cl | H | $CF_3$ | H | C-Cl | $-CH_2-CH-$<br>\|<br>$CH_3$ | $COOCH_3$ | Fp. 88 °C |
| 9 | IB | Cl | H | $CF_3$ | H | N | $-CH_2-CH-$<br>\|<br>Cl | $COOCH_3$ | δ:4,53(t)(-CH-)<br>\|<br>Cl |

Tabelle 3 - Fortsetzung

| Beisp.-Nr. | Isomeren-gruppe | $R^1$ | $R^2$ | $R^3$ | $R^4$ | X | Y | Z | Physikalische Daten |
|---|---|---|---|---|---|---|---|---|---|
| 10 | IB | Cl | H | $CF_3$ | H | N | $-CH_2-CH_2-$ | $COOCH_3$ | |
| 11 | IE | Cl | H | $CF_3$ | H | C-Cl | $-CH_2-CH-$<br>Cl | $COOCH_3$ | δ:4,66(t)(-CH-)<br>Cl |
| 12 | IE | Cl | H | $CF_3$ | H | C-Cl | $-CH_2-CH_2-$ | $COOCH_3$ | |
| 13 | IB | Cl | H | $CF_3$ | H | C-F | $-CH-CH_2-$<br>Br | $COOCH_3$ | δ:5,51(t)(-CH-)<br>Cl |

Ausgangsstoffe der Formel (II)

Beispiel (II-1)

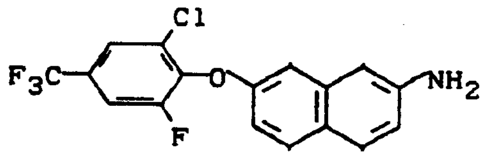

32,5 g (0,15 Mol) 3-Chlor-4,5-difluor-benzotrifluorid werden unter Rühren zu einer auf 120°C erwärmten Mischung aus 35,3 g (0,15 Mol) 7-Hydroxy-2-naphthylamin-Hydrochlorid, 20,1 g (0,36 Mol) Kaliumhydroxid (Pulver) und 300 ml Dimethylsulfoxid gegeben und das Reaktionsgemisch wird 20 Stunden bei 120°C gerührt. Dann wird bei 1 mbar eingeengt, der Rückstand in Essigsäureethylester gelöst, mit Wasser gewaschen, mit Natriumsulfat getrocknet und filtriert. Vom Filtrat wird das Lösungsmittel im Wasserstrahlvakuum sorgfältig abdestilliert.

Man erhält 42,7 g (80 %, der Theorie) 7-(2-Chlor-6-fluor-4-trifluormethyl-phenoxy)-2-naphthylamin als öligen Rückstand.

$^1$H-NMR ($CDCl_3$, δ): 6,85 (d), 6,95 (d), 7,60 (d).

Analog erhält man:

Beispiel (II-2)

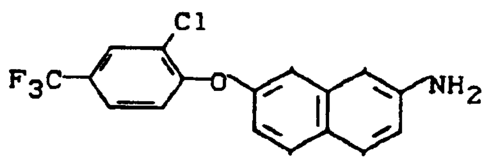

Schmelzpunkt: 115°C.

Beispiel (II-3)

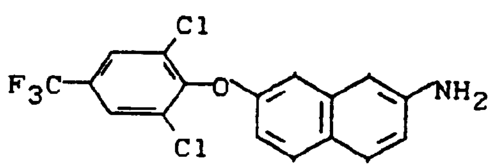

$^1$H-NMR (DMSO, δ): 8,15 (s)

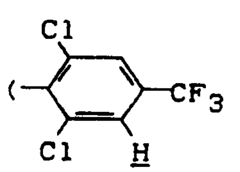
.

Beispiel (II-4)

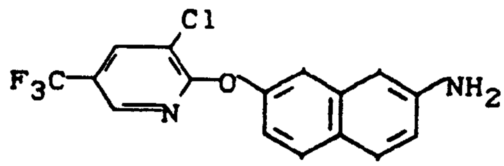

Schmelzpunkt: 121°C.

Anwendungsbeispiele

In den folgenden Anwendungsbeispielen wird die nachstehend aufgeführte Verbindung als Vergleichssubstanz herangezogen:

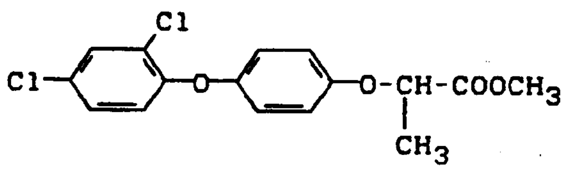

(A)

α-[4-(2,4-Dichlor-phenoxy)-phenoxy]-propionsäure-methylester (Diclofop-methyl)
(bekannt aus DE-OS 22 23 894/Beispiel 86).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt des Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirketoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden, nach drei Wochen wird der Schädigungsgrad der Pflanzen bonitiert in % Schädigung in Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in dar wirksamkeit gegenüber der Vergleichssubstanz (A) zeigen in diesem Test z.B. die Verbindungen gemäß Heretellungsbeispiel (1), (2), (3), (7), (8) und (11).

**Patentansprüche**

1. (Hetero)Aryloxynaphthalinderivate der Formel (I)

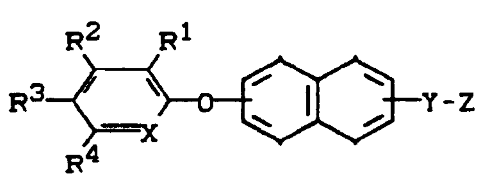

(I)

in welcher

$R^1$ für Wasserstoff, Halogen, Cyano oder Trifluormethyl steht,

$R^2$ für Wasserstoff oder Halogen steht,

$R^3$ für Halogen, Trifluormethyl, Trifluormethoxy, Trifluormethylthio oder Trifluormethylsulfonyl steht,

$R^4$ für Wasserstoff oder Halogen steht,

X für Stickstoff oder die Gruppierung C-$R^5$ steht, worin

$R^5$ für Wasserstoff oder Halogen steht,

Y für jeweils gegebenenfalls verzweigtes und/oder gegebenenfalls durch Halogen substituiertes Alkandiyl oder Alkendiyl, jeweils mit wenigstens 2 Kohlenstoffatomen, steht und

Z für Cyano oder die Gruppierung -CO-$Z^1$ steht, worin

$Z^1$ für Halogen, Hydroxy, amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung -O-$R^6$ steht, worin

$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Arylthioalkyl, Arylalkoxyalkyl, Arylalkylthioalkyl, Trialkylsilylalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl, Alkylidenamino oder für ein ammonium-, Alkylammonium-, Alkalimetall-oder Erdalkalimetalläquivalent steht oder für die Gruppierung

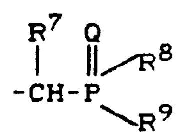

steht, worin

$R^7$ für Wasserstoff, Alkyl, Aryl, Furyl, Thienyl oder Pyridyl steht,

$R^8$ für Alkyl oder Alkoxy steht,

$R^9$ für Alkoxy steht und

Q für Sauerstoff oder Schwefel steht,

oder

$R^6$ weiterhin für die Gruppierung $-(CH_2)_n-R^{10}$ steht, worin

$R^{10}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und

n für die Zahlen 0, 1 oder 2 steht.

2. (Hetero)Aryloxynaphthalinderivate der Formel (I) gemäß Anspruch 1, in welcher

$R^1$ für Wasserstoff, Fluor, Chlor, Brom, Cyano oder Trifluormethyl steht,

$R^2$ für Wasserstoff, Fluor oder Chlor steht,

$R^3$ für Fluor, Chlor, Brom, Trifluormethyl, Trifluormethoxy, Trifluormethylthio oder , Trifluormethylsulfonyl steht,

$R^4$ für Wasserstoff, Fluor oder Chlor steht,

X für Stickstoff oder die Gruppierung C-$R^5$ steht, worin

$R^5$ für Wasserstoff, Fluor, Chlor oder Brom steht,

Y für jeweils gegebenenfalls verzweigtes und/oder gegebenenfalls durch Fluor, Chlor und/oder Brom substituiertes Alkandiyl oder Alkendiyl, jeweils mit 2 bis 4 Kohlenstoffatomen, steht und

Z für Cyano oder die Gruppierung -CO-$Z^1$ steht, worin

$Z^1$ für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^6$ steht, worin

$R^6$ für einen gegebenenfalls durch Fluor und/ oder Chlor substituierten Rest aus der Reihe

$C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_4$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Phenylaminocarbonyl-$C_1$-$C_4$-alkyl, N-($C_1$-$C_4$-Alkyl)-N-phenyl-aminocarbonyl-$C_1$-$C_4$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium-, ein Natrium-, Kalium-oder Calcium-äquivalent steht, oder für die Gruppierung

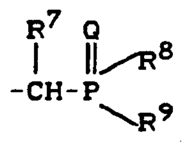

steht, worin

$R^7$ für Wasserstoff, $C_1$-$C_4$-Alkyl, Phenyl, Furyl, Thienyl oder Pyridyl steht,
$R^8$ für $C_1$-$C_4$-Alkyl oder $C_1$-$C_4$-Alkoxy steht,
$R^9$ für $C_1$-$C_4$-Alkoxy steht und
Q für Sauerstoff oder Schwefel steht,

oder

$R^6$ weiterhin für die Gruppierung $-(CH_2)_n-R^{10}$ steht, worin

n für die Zahlen 0, 1 oder 2 steht und
$R^{10}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

3. Verfahren zur Herstellung von (Hetero)Aryloxynaphthalinderivaten der Formel (I)

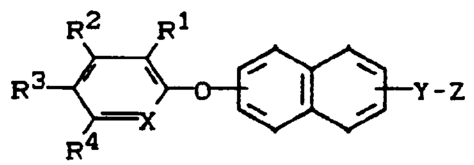

(I)

in welcher

$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht,
X für Stickstoff oder die Gruppierung C-$R^5$ steht, worin
$R^5$ für Wasserstoff oder Halogen steht,
Y für jeweils gegebenenfalls verzweigtes und/ oder gegebenenfalls durch Halogen substituiertes Alkandiyl oder Alkendiyl, jeweils mit wenigstens 2 Kohlenstoffatomen, steht und
Z für Cyano oder die Gruppierung -CO-$Z^1$ steht, worin
$Z^1$ für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonyl-hydrazino, Alkylthio, Arylthio, Aralkylthio, Alkoxycarbonylalkylthio oder für die Gruppierung -O-$R^6$ steht, worin
$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Arylthioalkyl, Arylalkoxyalkyl, Arylalkylthioalkyl, Trialkylsilylalkyl, Alkoxycarbonylalkyl, Alkylaminocarboxylalkyl, Arylaminocarbonylalkyl, N-Alkyl-N-aryl-aminocarbonylalkyl, Aralkyl, Azolylalkyl, Alkylidenamino oder für ein Ammonium-, Alkylammonium-, Alkalimetall-oder Erdalkalimetall-äquivalent steht oder für die Gruppierung

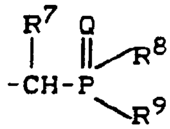

steht, worin

$R^7$ für Wasserstoff, Alkyl, Aryl, Furyl, Thienyl oder Pyridyl steht,

$R^8$ für Alkyl oder Alkoxy steht,

$R^9$ für Alkoxy steht und

Q für Sauerstoff oder Schwefel steht,

oder

$R^6$ weiterhin für die Gruppierung $-(CH_2)_n-R^{10}$ steht, worin

$R^{10}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und

n für die Zahlen 0, 1 oder 2 steht, dadurch gekennzeichnet, daß man

(a) (Hetero)Aryloxynaphthylamine der allgemeinen Formel (II)

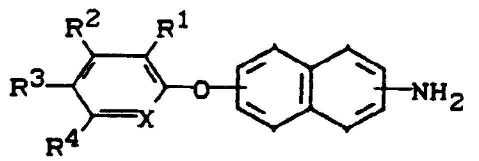

(II)

in welcher

$R^1$, $R^2$, $R^3$, $R^4$ und X die oben angegebenen Bedeutungen haben,

oder Säureaddukte von Verbindungen der Formel (II)

mit Natriumnitrit oder Kaliumnitrit und mit einem Hydrogenhalogenid ($HX^1$) in Gegenwart von Wasser und gegebenenfalls in Gegenwart eines organischen Lösungsmittels umsetzt und die dabei gebildeten Diazoniumsalze der allgemeinen Formel (III)

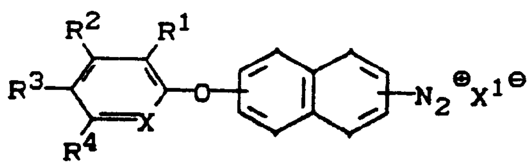

(III)

in welcher

$R^1$, $R^2$, $R^3$, $R^4$ und X die oben angegebenen Bedeutungen haben und

$X^1$ für Halogen steht,

mit Acrylsäurederivaten der allgemeinen Formel (IV)

$$Y^1 - Z^2 \quad \text{(IV)}$$

in welcher

$Y^1$ für gegebenenfalls verzweigtes und/oder gegebenenfalls durch Halogen substituiertes Alkenyl steht und

$Z^2$ für Cyano, Carboxy oder Alkoxycarbonyl steht,

in Gegenwart von Hydrogenhalogoniden ($HX^1$), gegebenenfalls in Gegenwart von Katalysatoren und gegebenenfalls in Gegenwart von Wasser und dem gegebenenfalls bei der Herstellung der Verbindungen der Formel (III) verwendeten organischen Lösungsmittel umsetzt und gegebenenfalls on den so erhaltenen Vorbindungen der Formel (I) weitere Derivatisierungen in dem durch obige Substituentendefinition vorgegebenen Rahmen durchführt, oder

(b) für den Fall, daß in Formel (I) Y für gegebenenfalls verzweigtes Alkandiyl steht und $R^1$, $R^2$, $R^3$, $R^4$, X und Z die oben angegebenen Bedeutungen haben,

Verbindungen der Formel (I), in welcher Y für gegebenenfalls verzweigtes und wenigstens einfach durch Halogen substituiertes Alkandiyl oder für gegebenenfalls durch Halogen substituiertes Alkendiyl steht sowie $R^1$, $R^2$, $R^3$, $R^4$, X und Z die oben angegebenen Bedeutungen haben, mit Wasserstoff in Gegenwart eines Hydrierungskatalysators und in Gegenwart eines Verdünnungsmittels umsetzt, oder

(c) für den Fall, daß in Formel (I) Y für durch Halogen substituiertes Alkandiyl steht und $R^1$, $R^2$, $R^3$, $R^4$, X und Z die oben angegebenen Bedeutungen haben,

Verbindungen der Formel (I), in welcher Y für Alkandiyl oder Alkendiyl steht und $R^1$, $R^2$, $R^3$, $R^4$, X und Z die oben angegebenen Bedeutungen haben,

mit Halogenierungsmitteln gegebenenfalls in Gegenwart von Katalysatoren und gegebenenfalls in Gegenwart von Verdünnungsmitteln umsetzt, oder

(d) für den Fall, daß in Formel (I) Z für die Gruppierung -CO-$Z^1$ steht, worin $Z^1$ für Hydroxy steht, und $R^1$, $R^2$, $R^3$, $R^4$, X und Y die oben angegebenen Bedeutungen haben,

Verbindungen der Formel (I), in welcher Z für Cyano oder die Gruppierung -CO-$Z^1$ steht, worin $Z^1$ für Methoxy oder Ethoxy steht, und $R^1$, $R^2$, $R^3$, $R^4$, X und Y die oben bezeichneten Bedeutungen haben,

mit Wasser, gegebenenfalls in Gegenwart eines Verseifungshilfsmittels und gegebenenfalls in Gegenwart eines organischen Lösungsmittels umsetzt, oder

(e) für den Fall, daß in Formel (I) Z für die Gruppierung -CO-$Z^1$ steht, worin $Z^1$ für Halogen steht, und $R^1$, $R^2$, $R^3$, $R^4$, X und Y die oben angegebenen Bedeutungen haben,

Verbindungen der Formel (I), in welcher Z für die Gruppierung -CO-$Z^1$ steht, worin $Z^1$ für Hydroxy steht, und $R^1$, $R^2$, $R^3$, $R^4$, X und Z die oben angegebenen Bedeutungen haben,

mit einem Halogenierungsmittel, gegebenenfalls in Gegenwart eines Katalysators und gegebenenfalls in Gegenwart eines Verdünnungsmittels, umsetzt, oder

(f) für den Fall, daß in Formel (I) Z für die Gruppierung -CO-$Z^1$ steht, worin $Z^1$ mit Ausnahme von Halogen die oben angegebene Bedeutung hat, und $R^1$, $R^2$, $R^3$, $R^4$, X und Y die oben angegebenen Bedeutungen haben,

Verbindungen der Formel (I), in welcher Z für die Gruppierung -CO-$Z^1$ steht, worin $Z^1$ für Halogen steht, und $R^1$, $R^2$, $R^3$, $R^4$, X und Y die oben angegebenen Bedeutungen haben,

mit Verbindungen der allgemeinen Formel (V)

$$H - Z^1 \quad (V)$$

in welcher

$Z^1$ mit Ausnahme von Halogen die oben angegebene Bedeutung hat,

gegebenenfalls in Gegenwart eines Säureakzeptors und gegebenenfalls in Gegenwart eines Verdünnungsmittels umsetzt, oder

(g) für den Fall, daß in Formel (I) Y für gegebenenfalls verzweigtes und/oder gegebenenfalls durch Halogen substituiertes Alkendiyl steht, und $R^1$, $R^2$, $R^3$, $R^4$, X und Z die oben angegebenen Bedeutungen haben,

Verbindungen der Formel (I), in welcher Y für wenigstens einfach durch Halogen substituiertes Alkandiyl mit wenigstens 2 Kohlenstoffatomen steht, und $R^1$, $R^2$, $R^3$, $R^4$, X und Z die oben angegebenen Bedeutungen haben,

mit Basen gegebenenfalls in Gegenwart von Verdünnungsmitteln umsetzt.

4. Herbizide Mittel, gekennzeichnet durch einen Gehalt an mindestens einem (Hetero)Aryloxynaphthalinderivat der Formel (I) gemäß den Ansprüchen 1 bis 3.

5. Verfahren zur Bekämpfung von Unkräutern, dadurch gekennzeichnet, daß man (Hetero)Aryloxynaphthalinderivate der Formel (I) gemäß den Ansprüchen 1 bis 3 auf die Unkräuter und/oder ihren Lebensraum einwirken läßt.

6. Verwendung von (Hetero)Aryloxynaphthalinderivaten der Formel (I) gemäß den Ansprüchen 1 bis 3 zur Bekämpfung von Unkräutern.

7. Verfahren zur Herstellung von herbiziden Mitteln, dadurch gekennzeichnet, daß man (Hetero)Aryloxynaphthalinderivate der Formel (I) gemäß den Ansprüchen 1 bis 3 mit Streckmitteln und/oder oberflächenaktiven Substanzen vermischt.

8. (Hetero)Aryloxynaphthylamine der Formel (II)

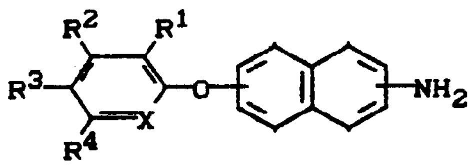

(II)

in welcher
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht und
X für Stickstoff oder die Gruppierung C-$R^5$ ateht, worin
$R^5$ für Wasserstoff oder Halogen steht.

9. Verfahren zur Herstellung von (Hetero)Aryloxynaphthylaminen der Formel (II)

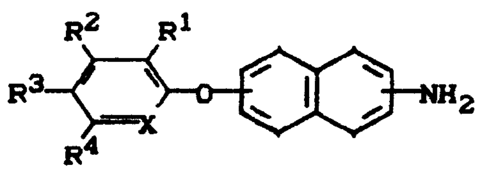

(II)

in welcher
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht und
X für Stickstoff oder die Gruppierung C-$R^5$ steht, worin
$R^5$ für Wasserstoff oder Halogen steht.
dadurch gekennzeichnet, daß man Halogen(hetero)arylverbindungen der Formel (VI)

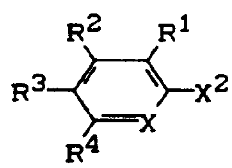

(VI)

in welcher
$R^1$, $R^2$, $R^3$, $R^4$ und X die oben angegebenen Bedeutungen haben und
$X^2$ für Fluor oder Chlor steht,
mit Hydroxynaphthylaminen der allgemeinen Formel (VII)

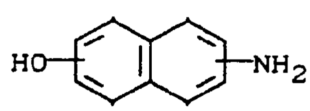

(VII)

oder mit deren Hydrogenhalogeniden,
in Gegenwart eines Säureakzeptors und in Gegenwart eines Verdünnungsmittels umsetzt.

## Claims

1. (Hetero)aryloxynaphthalene derivatives of the formula (I)

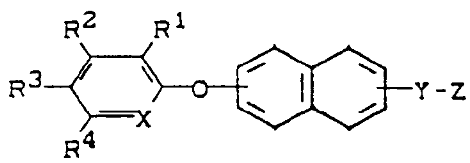

(I)

in which
$R^1$ represents hydrogen, halogen, cyano or trifluoromethyl,
$R^2$ represents hydrogen or halogen,
$R^3$ represents halogen, trifluoromethyl, trifluoromethoxy, trifluoromethylthio or trifluoromethylsulphonyl,
$R^4$ represents hydrogen or halogen,
X represents nitrogen or the C-$R^5$ group,
where
$R^5$ represents hydrogen or halogen,
Y represents in each case optionally branched and/or optionally halogen-substituted alkanediyl or alkenediyl, in each case having at least 2 carbon atoms, and
Z represents cyano or the -CO-$Z^1$ group, where
$Z^1$ represents halogen, hydroxyl, amino, alkylamino, alkenylamino, alkinylamino, arylamino, aralkylamino, alkoxycarbonylalkylamino, cyanoamino, dialkylamino, dialkenylamino, alkylsulphonylamino,
arylsulphonylamino, hydroxyamino, alkoxy amino,hydrazino,alkylsulphonylhydrazino, arylsulphonylhydrazino, alkylthio, arylthio, aralkylthio or alkoxycarbonylalkylthio, or represents the -O-$R^6$ group, where
$R^6$ represents an optionally halogensubstituted radical from the series comprising alkyl, alkenyl, alkinyl, alkoxyalkyl, alkylthioalkyl, alkylsulphinylalkyl, alkylsulphonyl alkyl, aryloxyalkyl, arylthioalkyl, arylalkoxyalkyl, arylalkylthioalkyl, trialkylsilylalkyl, alkoxycarbonyl alkyl,alkylaminocarbonylalkyl, arylaminocarbonylalkyl, N-alkyl-N-aryl-aminocarbonylalkyl, aralkyl,azolylalkyl or alkylideneamino, or represents an ammonium, alkylammonium, alkali metal or alkaline earth metal equivalent, or represents the

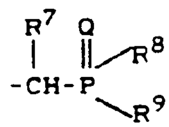

group where,
$R^7$ represents hydrogen, alkyl, aryl, furyl, thienyl or pyridyl,
$R^8$ represents alkyl or alkoxy,
$R^9$ represents alkoxy and
Q represents oxygen or sulphur,
or
$R^6$ furthermore represents the $-(CH_2)_nR^{10}$ group, where
$R^{10}$ represents an optionally halogen- and/or alkyl-substituted heterocyclic radical from the series comprising furyl, tetrahydrofuryl, oxotetrahydrofuryl, thienyl, tetrahydrothienyl, perhydropyranyl, oxazolyl, thiazolyl, thiadiazolyl, dioxolanyl, perhydropyrrolyl, oxoperhydropyrrolyl, pyridinyl or pyrimidinyl, and
n represents the numbers 0, 1 or 2.

2. (Hetero)aryloxynaphthalene derivatives of the formula (I) according to Claim 1, in which
$R^1$ represents hydrogen, fluorine, chlorine, bromine, cyano or trifluoromethyl,
$R^2$ represents hydrogen, fluorine or chlorine,
$R^3$ represents fluorine, chlorine, bromine, trifluoromethyl, trifluoromethoxy, trifluoromethylthio or trifluoromethylsulphonyl,

$R^4$ represents hydrogen, fluorine or chlorine,

X represents nitrogen or the C-$R^5$ group, where

$R^5$ represents hydrogen, fluorine, chlorine or bromine,

Y represents in each case optionally branched and/or optionally fluorine-, chlorine- and/or bromine-substituted alkanediyl or alkenediyl, in each case having 2 to 4 carbon atoms, and

Z represents cyano or the -CO-$Z^1$ group, where

$Z^1$ represents chlorine, hydroxyl, amino, $C_1$-$C_6$-alkylamino, $C_3$-$C_4$-alkenylamino, $C_3$-$C_4$-alkinylamino, phenylamino, benzylamino, $C_1$-$C_4$-alkoxycarbonyl-$C_1$-$C_2$-alkylamino, cyanoamino, di- ($C_1$-$C_4$-alkyl ) -amino, di- ( $C_3$-$C_4$-alkenyl ) -amino, $C_1$-$C_4$-alkylsulphonylamino, phenylsulphonylamino, tolylsulphonylamino, hydroxyamino, $C_1$-$C_6$-alkoxyamino, hydrazino, $C_1$-$C_4$-alkylsulphonylhydrazino, phenylsulphonylhydrazino, tolylsulphonylhydrazino, $C_1$-$C_4$-alkylthio, phenylthio, benzylthio or $C_1$-$C_2$-alkoxycarbonyl-$C_1$-$C_4$-alkylthio, or represents the -O-$R^6$ group, where

$R^6$ represents an optionally fluorine and/or chlorine-substituted radical from the series comprising $C_1$-$C_6$-alkyl, $C_3$-$C_4$-alkenyl, $C_3$-$C_4$-alkinyl, $C_1$-$C_4$-alkoxy-$C_1$-$C_4$-alkyl, $C_1$-$C_4$-alkylthio-$C_1$-$C_4$-alkyl, $C_1$-$C_4$-alkylsulphinyl-$C_1$-$C_4$-alkyl, $C_1$-$C_4$-alkylsulphonyl-$C_1$-$C_4$-alkyl, phenoxy-$C_1$-$C_3$-alkyl, trimethylsilylmethyl, phenylthio-$C_1$-$C_3$-alkyl, benzyloxy-$C_1$-$C_3$-alkyl, benzylthio-$C_1$-$C_3$-alkyl, $C_1$-$C_4$-alkoxy-carbonyl-$C_1$-$C_2$-alkyl, $C_1$-$C_4$-alkylamino-carbonyl-$C_1$-$C_2$-alkyl, phenylaminocarbonyl-$C_1$-$C_4$-alkyl, N-( $C_1$-$C_4$-alkyl ) -N-phenyl-aminocarbonyl-$C_1$-$C_4$-alkyl, benzyl, pyrazolyl-$C_1$-$C_4$-alkyl or $C_2$-$C_4$-alkylideneamino, or represents an ammonium, a $C_1$-$C_4$-alkylammonium, a sodium, potassium or calcium equivalent, or represents the

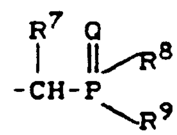

group, where

$R^7$ represents hydrogen, $C_1$-$C_4$-alkyl, phenyl, furyl, thienyl or pyridyl,

$R^8$ represents $C_1$-$C_4$-alkyl or $C_1$-$C_4$-alkoxy,

$R^9$ represents $C_1$-$C_4$-alkoxy and

Q represents oxygen or sulphur,

or

$R^6$ furthermore represents the -$(CH_2)_n R^{10}$ group, where

n represents the numbers 0, 1 or 2 and

$R^{10}$ represents an optionally fluorine-, chlorine-, bromine- and/or $C_1$-$C_4$-alkyl-substituted heterocyclic radical from the series comprising furyl, tetrahydrofuryl, oxotetrahydrofuryl, thienyl, tetrahydrothienyl, perhydropyranyl, oxazolyl, thiazolyl,thiadiazolyl,dioxolanyl, perhydropyrrolyl, oxoperhydropyrrolyl, pyridinyl or pyrimidinyl.

3. Process for the preparation of (hetero)aryloxynaphthalene derivatives of the formula (I)

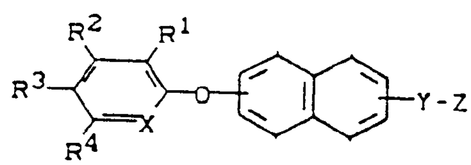

(I)

in which

$R^1$ represents hydrogen, halogen, cyano or trifluoromethyl,

$R^2$ represents hydrogen or halogen,

$R^3$ represents halogen, trifluoromethyl, trifluoromethoxy, trifluoromethylthio or trifluoromethylsulphonyl,

$R^4$ represents hydrogen or halogen,

X represents nitrogen or the C-$R^5$ group, where

$R^5$ represents hydrogen or halogen,

Y represents in each case optionally branched and/or optionally halogen-substituted alkanediyl or alkenediyl, in each case having at least 2 carbon atoms, and

Z represents cyano or the -CO-$Z^1$ group, where

$Z^1$ represents halogen, hydroxyl, amino, alkylamino, alkenylamino, alkinylamino, arylamino,aralkylamino,alkoxycarbonylalkylamino, cyanoamino, dialkylamino, dialkenylamino, alkylsulphonylamino, arylsulphonylamino,hydroxyamino,alkoxyamino, hydrazino, alkylsulphonylhydrazino, arylsulphonylhydrazino, alkylthio, arylthio, aralkylthio or alkoxycarbonylalkylthio, or represents the -O-$R^6$ group, where

$R^6$ represents an optionally halogen-sub stituted radical from the series comprising alkyl, alkenyl, alkinyl, alkoxyalkyl, alkylthioalkyl, alkylsul phinylalkyl, alkylsulphonylalkyl, aryloxyalkyl, arylthioalkyl, arylalko xyalkyl, arylalkylthioalkyl, trialkyl silylalkyl, alkoxycarbonylalkyl, alkylaminocarbonylalkyl, arylaminocar bonylalkyl, N-alkyl-N-aryl-aminocar bonylalkyl, aralkyl, azolylalkyl or alkylideneamino, or represents an am monium, alkylammonium, alkali metal or alkaline earth metal equivalent, or represents the

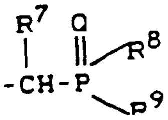

group, where

$R^7$ represents hydrogen, alkyl, aryl, furyl, thienyl or pyridyl,

$R^8$ represents alkyl or alkoxy,

$R^9$ represents alkoxy and

Q represents oxygen or sulphur, or

$R^6$ furthermore represents the $-(CH_2)_nR^{10}$ group, where

$R^{10}$ represents an optionally halogen- and/or alkyl-substituted heterocyclic radical from the series comprising furyl, tetrahydrofuryl, oxotetrahydrofuryl, thienyl, tetrahydrothienyl, perhydropyranyl, oxazolyl, thiazolyl, thiadiazolyl, dioxolanyl, perhydropyrrolyl, oxoperhydropyrrolyl, pyridinyl or pyrimidinyl, and

n represents the numbers 0, 1 or 2,

characterized in that

(a) (hetero)aryloxynaphthylamines of the general formula (II)

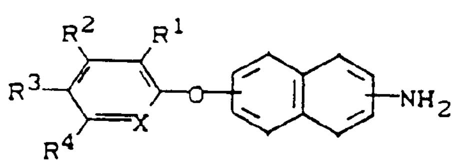

(II)

in which

$R^1$, $R^2$, $R^3$, $R^4$ and X have the abovementioned meanings,

or acid adducts of compounds of the formula (II)

are reacted with sodium nitrite or potassium nitrite and with a hydrogen halide ($HX^1$) in the presence of water and if appropriate in the presence of an organic solvent, and the diazonium salts which are formed in this process, of the general formula (III)

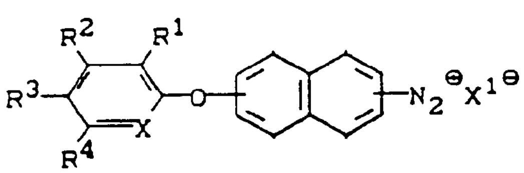

(III)

in which
$R^1$, $R^2$, $R^3$, $R^5$ and X have the abovementioned meanings and
$X^1$ represents halogen,
are reacted with acrylic acid derivatives of the general formula (IV)

$$Y^1 - Z^2 \quad (IV)$$

in which
$Y^1$ represents optionally branched and/or optionally halogen-substituted alkenediyl and
$Z^2$ represents cyano, carboxyl or alkoxycarbonyl,
in the presence of hydrogen halides ($HX^1$), if appropriate in the presence of catalysts and if appropriate in the presence of water and the organic solvent which, if appropriate, has been used in the preparation of the compounds of the formula (III), and, if appropriate, the formation of other derivatives is carried out on the resulting compounds of the formula (I) within the scope predetermined by the above definition of substituents, or

(b) in the event that Y in formula (I) represents optionally branched alkanediyl and $R^1$, $R^2$, $R^3$, $R^4$, X and Z have the abovementioned meanings,
compounds of the formula (I) in which Y represents optionally branched alkanediyl which is at least monosubstituted by halogen, or represents optionally halogen-substituted alkenediyl, and $R^1$, $R^2$, $R^3$, $R^4$, X and Z have the abovementioned meanings,
are reacted with hydrogen in the presence of a hydrogenation catalyst and in the presence of a diluent, or

(c) in the event that Y in formula (I) represents halogen-substituted alkanediyl and $R^1$, $R^2$, $R^3$, $R^4$, X and Z have the abovementioned meanings,
compounds of the formula (I) in which Y represents alkanediyl or alkenediyl and $R^1$, $R^2$, $R^3$, $R^4$, X and Z have the abovementioned meanings,
are reacted with halogenating agents, if appropriate in the presence of catalysts and if appropriate in the presence of diluents, or

(d) in the event that Z in formula (I) represents the $-CO-Z^1$ group, where $Z^1$ represents hydroxyl, and $R^1$, $R^2$, $R^3$, $R^4$, X and Y have the abovementioned meanings,
compounds of the formula (I) in which Z represents cyano or the $-CO-Z^1$ group, where $Z^1$ represents methoxy or ethoxy, and $R^1$, $R^2$, $R^3$, $R^4$, X and Y have the meanings specified above,
are reacted with water, if appropriate in the presence of a hydrolyzation auxiliary and if appropriate in the presence of an organic solvent, or

(e) in the event that Z in formula (I) represents the $-CO-Z^1$ group, where $Z^1$ represents halogen, and $R^1$, $R^2$, $R^3$, $R^4$, X and Y have the abovementioned meanings,
compounds of the formula (I) in which Z represents the $-CO-Z^1$ group, where $Z^1$ represents hydroxyl, and $R^1$, $R^2$, $R^3$, $R^4$, X and Z have the abovementioned meanings,
are reacted with a halogenating agent, if appropriate in the presence of a catalyst and if appropriate in the presence of a diluent, or

(f) in the event that Z in formula (I) represents the $-CO-Z^1$ group, where $Z^1$ has the abovementioned meaning with the exception of halogen, and $R^1$, $R^2$, $R^3$, $R^4$, X and Y have the abovementioned meanings,
compounds of the formula (I) in which Z represents the $-CO-Z^1$ group, where $Z^1$ represents halogen, and $R^1$, $R^2$, $R^3$, $R^4$, X and Y have the abovementioned meanings,
are reacted with compounds of the general formula (V)

$$H - Z^1 \quad (V)$$

in which
$Z^1$ has the abovementioned meaning with the exception of halogen,
if appropriate in the presence of an acid acceptor and if appropriate in the presence of a diluent, or

(g) in the event that Y in formula (I) represents optionally branched and/or optionally halogensubstituted alkenediyl, and $R^1$, $R^2$, $R^3$, $R^4$, X and Z have the abovementioned meanings,
compounds of the formula (I) in which Y represents alkanediyl which has at least 2 carbon atoms and which is at least monosubstituted by halogen, and $R^11$, $^2$, $R^3$, $R^4$, X and Z have the abovementioned meanings,
are reacted with bases, if appropriate in the presence of diluents.

**4.** Herbicidal agents, characterized in that they contain at least one (hetero)aryloxynaphthalene derivative of the formula (I) according to Claims 1 to 3.

5. Method of combating weeds, characterized in that (hetero)aryloxynaphthalene derivatives of the formula (I) according to Claims 1 to 3 are allowed to act on the weeds and/or their environment.

6. Use of (hetero)aryloxynaphthalene derivatives of the formula (I) according to Claims 1 to 3 for combating weeds.

7. Process for the preparation of herbicidal agents, characterized in that (hetero)aryloxynaphthalene derivatives of the formula (I) according to Claims 1 to 3 are mixed with extenders and/or surface-active substances.

8. (Hetero)aryloxynaphthylamines of the formula (II)

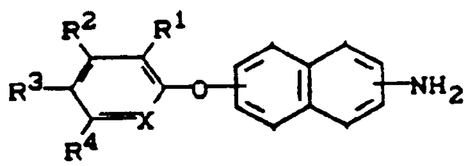

(II)

in which
$R^1$ represents hydrogen, halogen, cyano or trifluoromethyl,
$R^2$ represents hydrogen or halogen,
$R^3$ represents halogen, trifluoromethyl, trifluoromethoxy, trifluoromethylthio or trifluoromethylsulphonyl,
$R^4$ represents hydrogen or halogen and
X represents nitrogen or the C-$R^5$ group, where
$R^5$ represents hydrogen or halogen.

9. Process for the preparation (hetero)aryloxynaphthylamines of the formula (II)

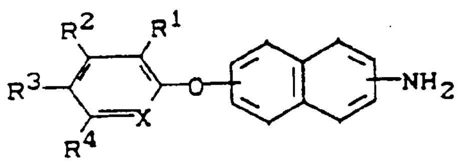

(II)

in which
$R^1$ represents hydrogen, halogen, cyano or trifluoromethyl,
$R^2$ represents hydrogen or halogen,
$R^3$ represents halogen, trifluoromethyl, trifluoromethoxy, trifluoromethylthio or trifluoromethylsulphonyl,
$R^4$ represents hydrogen or halogen,
X represents nitrogen or the C-$R^5$ group, where
$R^5$ represents hydrogen or halogen, characterized in that halogeno(hetero)aryl compounds of the formula (VI)

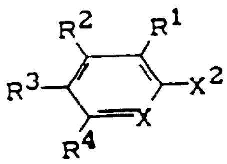

(VI)

in which
$R^1$, $R^2$, $R^3$, $R^4$ and X have the abovementioned meanings and
$X^2$ represents fluorine or chlorine,

are reacted with hydroxynaphthylamines of the general formula (VII)

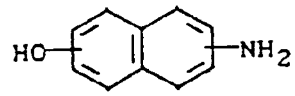

(VII)

or with their hydrogen halides,
in the presence of an acid acceptor and in the presence of a diluent.

**Revendications**

1. Dérivé (hétéro)aryloxynaphtaléniques de formule (I)

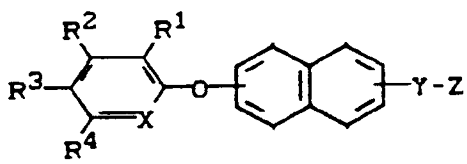

(I)

dans laquelle

$R^1$ représente l'hydrogène, un halogène, un groupe cyano ou trifluorométhyle,

$R^2$ est l'hydrogène ou un halogène,

$R^3$ est un halogène, un groupe trifluorométhyle, trifluorométhoxy, trifluorométhylthio ou trifluorométhylsulfonyle,

$R^4$ est l'hydrogène ou un halogène,

X représente l'azote ou le groupement C-$R^5$, où

$R^5$ est l'hydrogène ou un halogène,

Y est un groupe alcanediyle ou un groupe alcènediyle, chacun éventuellement ramifié et/ou éventuellement substitué par un halogène, avec chacun au moins 2 atomes de carbone et

Z est un groupe cyano ou le groupement -CO-$Z^1$,

dans lequel

$Z^1$ est un halogène, un groupe hydroxy, amino, alkylamino, alcénylamino, alcynylamino, arylamino, aralkylamino, alkoxycarbonylalkylamino, cyanamino, dialkylamino, dialcénylamino, alkylsulfonylamino, arylsulfonylamino, hydroxyamino, alkoxyamino, hydrazino, alkylsulfonyldrazino, arylsulfonylhydrazino, alkylthio, arylthio, aralkylthio, alkoxycarbonylalkylthio ou le groupement -O-$R^6$, dans lequel

$R^6$ est un reste éventuellement substitué par un halogène, de la série alkyle, alcényle, alcynyle, alkoxyalkyle, alkylthioalkyle, alkylsulfinylalkyle, alkylsulfonyalkyle, aryloxyalkyle, arylthioalkyle, arylalkoxyalkyle, arylalkylthioalkyle, trialkylsilylalkyle, alkoxycarbonylalkyle, akylaminocarbonylalkyle, arylaminocarbonylalkyle, N-alkyl-N-arylaminocarbonylalkyle, aralkyle, azolylalkyle, alkylidèneamino ou un équivalent d'ammonium,d'alkylammonium, de métal alcalin ou de métal alcalinoterreux ou représente le groupement

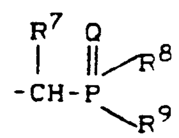

,

dans lequel

$R^7$ est l'hydrogène, un groupe alkyle, aryle, furyle, thiényle ou pyridyle,

$R^8$ est un groupe alkyle ou alkoxy,

$R^9$ est un groupe alkoxy et

Q représente l'oxygène ou le soufre,

ou bien

$R^6$ représente en outre le groupement $-(CH_2)_n-R^{10}$, dans lequel

$R^{10}$ est un reste hétérocyclique portant éventuellement un substituant halogéno et/ou alkyle, de la série furyle, tétrahydrofuryle, oxotétrahydrofuryle, thiényle, tétrahydrothiényle, perhydropyrannyle, oxazolyle, thiazolyle, thiadiazolyle, dioxolanyle, perhydropyrrolyle, oxoperhydropyrrolyle, pyridinyle ou pyrimidinyle et

n représente les nombres 0, 1 ou 2.

2. Dérivés (hétéro)aryloxynaphtaléniques de formule (I) suivant la revendication 1, dans laquelle

$R^1$ est l'hydrogène, le fluor, le chlore, le brome, un groupe cyano ou trifluorométhyle,

$R^2$ est l'hydrogène, le fluor ou le chlore,

$R^3$ est le fluor, le chlore, le brome, un groupe trifluorométhyle, trifluorométhoxy, trifluorométhylthio ou trifluorométhylsulfonyle,

$R^4$ est l'hydrogène, le fluor ou le chlore,

X est l'azote ou le groupement $C-R^5$, où

$R^5$ est l'hydrogène, le fluor, le chlore ou le brome,

Y désigne un groupe alcanediyle ou un groupe alcènediyle, chacun éventuellement ramifié et/ou éventuellement substitué par du fluor, du chlore et/ou du brome et chacun avec 2 à 4 atomes de carbone, et

Z est un groupe cyano ou le groupement $-CO-Z^1$,

dans lequel

$Z^1$ représente le chlore, un groupe hydroxy, amino, alkylamino en $C_1$ à $C_6$, alcénylamino en $C_3$ ou $C_4$, alcynylamino en $C_3$ ou $C_4$, phénylamino, benzylamino, (alkoxy en $C_1$ à $C_4$)-carbonyl-(alkylamino en $C_1$ ou $C_2$), cyanamino, di(alkyle en $C_1$ à $C_4$)amino, di(alcényle en $C_3$ ou $C_4$)amino, alkylsulfonylamino en $C_1$ à $C_4$, phénylsulfonylamino, tolylsulfonylamino, hydroxyamino, alkoxyamino en $C_1$ à $C_6$, hydrazino, alkylsulfonylhydrazino en $C_1$ à $C_4$, phénylsulfonylhydrazino, tolylsulfonylhydrazino, alkylthio en $C_1$à $C_4$, phénylthio, benzylthio, (alkoxy en $C_1$ à $C_4$)-carbonyl(alkylthio en $C_1$ ou $C_2$) ou le groupement $-O-R^6$, dans lequel

$R^6$ est un reste éventuellement substitué par du fluor et/ou par du chlore, de la série alkyle en $C_1$ à $C_6$, alcényle en $C_3$ ou $C_4$, alcynyle en $C_3$ ou $C_4$, (alkoxy en $C_1$ à $C_4$)-(alkyle en $C_1$ à $C_4$), (alkylthio en $C_1$ à $C_4$)-(alkyle en $C_1$ à $C_4$), (alkylsulfinyle en $C_1$ à $C_4$)-(alkyle en $C_1$ à $C_4$), (alkylsulfonyle en $C_1$ à $C_4$)(alkyle en $C_1$ à $C_4$), phénoxy(alkyle en $C_1$ à $C_3$), triméthylsilylméthyle, phénylthio-(alkyle en $C_1$ à $C_3$), benzyloxy-(alkyle en $C_1$ à $C_3$), benzylthio-(alkyle en $C_1$ à $C_3$), (alkoxy en $C_1$ à $C_4$)carbonyl-(alkyle en $C_1$ ou $C_2$), (alkylamino en $C_1$ à $C_4$)-carbonyl-(alkyle en $C_1$ ou $C_2$), phénylaminocarbonyl-(alkyle en $C_1$ à $C_4$), N-(alkyle en $C_1$ à $C_4$)-N-phényl-aminocarbonyl(alkyle en $C_1$ à $C_4$), benzyle, pyrazolyl-(alkyle en $C_1$ à $C_4$), alkylidèneamino en $C_2$ à $C_4$ ou un équivalent d'ammonium, d'un (alkyle en $C_1$ à $C_4$)-ammonium, de sodium, de potassium ou de calcium ou le groupement

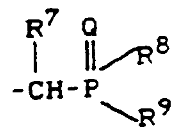

dans lequel

$R^7$ représente l'hydrogène, un groupe alkyle en $C_1$ à $C_4$, phényle, furyle, thiényle ou pyridyle,

$R^8$ est un groupe alkyle en $C_1$ à $C_4$ ou alkoxy en $C_1$ à $C_4$,

$R^9$ est un groupe alkoxy en $C_1$ à $C_4$ et

Q représente l'oxygène ou le soufre,

ou bien

$R^6$ représente en outre le groupement $-(CH_2)_n-R^{10}$, dans lequel

n représente les nombres 0, 1 ou 2 et

$R^{10}$ est un reste hétérocyclique éventuellement substitué par du fluor, du chlore, du brome et/ou un radical alkyle en $C_1$à $C_4$, de la série furyle, tétrahydrofuryle, oxotétrahydrofuryle, thiényle, tétrahydrothiényle, perhydropyrannyle, oxazolyle, thiazolyle, thiadiazolyle, dioxolanyle, perhydropyrrolyle, oxoperhydropyrrolyle, pyridinyle ou pyrimidinyle.

3. Procédé de production de dérivés (hétéro)aryloxynaphtaléniques de formule (I)

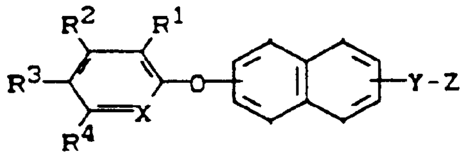 (I)

dans laquelle

$R^1$ représente l'hydrogène, un halogène, un groupe cyano ou trifluorométhyle,

$R^2$ est l'hydrogène ou un halogène,

$R^3$ est un halogène, un groupe trifluorométhyle, trifluorométhoxy, trifluorométhylthio ou trifluorométhylsulfonyle,

$R^4$ est l'hydrogène ou un halogène,

X représente l'azote ou le groupement C-$R^5$, où

$R^5$ est l'hydrogène ou un halogène,

Y est un groupe alcanediyle ou un groupe alcènediyle, chacun éventuellement ramifié et/ou éventuellement substitué par un halogène, avec chacun au moins 2 atomes de carbone et

Z est un groupe cyano ou le groupement -CO-$Z^1$,

dans lequel

$Z^1$ est un halogène, un groupe hydroxy, amino, alkylamino, alcénylamino, alcynylamino, arylamino, aralkylamino, alkoxycarbonylalkylamino, cyanamino, dialkylamino, dialcénylamino, alkylsulfonylamino, arylsulfonylamino, hydroxyamino, alkoxyamino, hydrazino, alkylsulfonylhydrazino, arylsulfonylhydrazino, alkylthio, arylthio, aralkylthio, alkoxycarbonylalkylthio ou le groupement -O-$R^6$, dans lequel

$R^6$ est un reste éventuellement substitué par un halogène, de la série alkyle, alcényle, alcynyle, alkoxyalkyle, alkylthioalkyle, alkylsulfinylalkyle, alkylsulfonylalkyle, aryloxyalkyle, arylthioalkyle, arylalkoxyalkyle, arylalkylthioalkyle, trialkylsilylalkyle, alkoxycarbonylalkyle, alkylaminocarbonylalkyle, arylaminocarbonylalkyle, N-alkyl-N-arylaminocarbonylalkyle, aralkyle, azolylalkyle, alkylidèneamino ou un équivalent d'ammonium, d'alkylammonium, de métal alcalin ou de métal alcalinoterreux ou représente le groupement

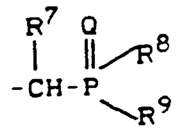 ,

dans lequel

$R^7$ est l'hydrogène, un groupe alkyle, aryle, furyle, thiényle ou pyridyle,

$R^8$ est un groupe alkyle ou alkoxy,

$R^9$ est un groupe alkoxy et

Q représente l'oxygène ou le soufre,

ou bien

$R^6$ représente en outre le groupement $-(CH_1)_n-R^{10}$, dans lequel

$R^{10}$ est un reste hétérocyclique portant éventuellement un substituant halogéno et/ou alkyle, de la série furyle, tétrahydrofuryle, oxotétrahydrofuryle, thiényle, tétrahydrothiényle, perhydropyrannyle, oxazolyle, thiazolyle, thiadiazolyle, dioxolanyle, perhydropyrrolyle, oxoperhydropyrrolyle, pyridinyle ou pyrimidinyle et

n représente les nombres 0, 1 ou 2,

caractérisé en ce que

(a) On fait réagir des (hétéro)aryloxynaphtylamines de formule générale (II)

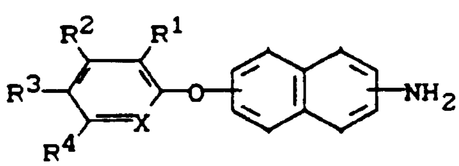 (II)

dans laquelle
$R^1$, $R^2$, $R^3$, $R^4$ et X ont les définitions indiquées ci-dessus
ou des produits d'addition d'acides de composés de formule (II)
avec le nitrite de sodium ou le nitrite de potassium et avec un halogénure d'hydrogène ($HX^1$) en présence d'eau et le cas échéant en présence d'un solvant organique et on fait réagir les sels de diazonium ainsi formés, de formule générale (III)

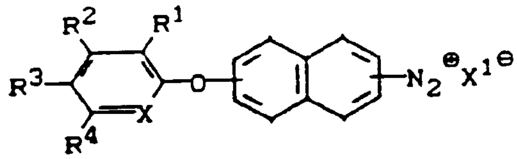
(III)

dans laquelle
$R^1$, $R^2$, $R^3$, $R^4$ et X ont les définitions indiquées ci-dessus et
$X^1$ est un halogène,
avec des dérivés d'acide acrylique de formule générale (IV)

$$Y^1 - Z^2 \quad (IV)$$

dans laquelle
$Y^1$ est un groupe alcényle éventuellement ramifié et/ou éventuellement substitué par un halogène et
$Z^2$ est un groupe cyano, carboxy ou alkoxycarbonyle,
en présence d'halogénures d'hydrogène ($HX^1$), le cas échéant en présence de catalyseurs et en la présence éventuelle d'eau et du solvant organique éventuellement utilisé dans la préparation des composés de formule (III) et on effectue éventuellement sur les composés de formule (I) ainsi obtenus d'autres dérivatisations dans le cadre prédéterminé dans la définition des substituants donnée ci-dessus, ou bien
(b) au cas ou dans la formule (I), Y représente un groupe alcanediyle éventuellement substitué et $R^1$, $R^2$, $R^3$, $R^4$, X et Z ont les définitions indiquées ci-dessus,
on fait réagir des composés de formule (I) dans laquelle Y est un groupe alcanediyle éventuellement ramifié et substitué au moins une fois par un halogène ou un groupe alcènediyle éventuellement substitué par un halogène de même que $R^1$, $R^2$, $R^3$, $R^4$, X et Z ont les définitions indiquées ci-dessus, avec l'hydrogène en présence d'un catalyseur d'hydrogénation et en présence d'un diluant, ou bien
(c) au cas ou dans la formule (I), Y est un groupe alcanediyle substitué par un halogène et $R^1$, $R^2$, $R^3$, $R^4$, X et Z ont les définitions indiquées ci-dessus,
on fait réagir des composés de formule (I) dans laquelle Y est un groupe alcanediyle ou un groupe alcènediyle et $R^1$, $R^2$, $R^3$, $R^4$, X et Z ont les définitions indiquées ci-dessus,
avec des agents d'halogénation, éventuellement en présence de catalyseurs et en la présence éventuelle de diluants, ou bien
(d) au cas ou dans la formule (I), Z représente le groupement -CO-$Z^1$, où $Z^1$ est un groupe hydroxy et $R^1$, $R^2$, $R^3$, $R^4$, X et Y ont les définitions indiquées ci-dessus, on fait réagir des composés de formule (I), dans laquelle Z est un groupe cyano ou le groupement -CO-$Z^1$, où $Z^1$ est un groupe méthoxy ou éthoxy et $R^1$, $R^2$, $R^3$, $R^4$, X et Y ont les définitions indiquées ci-dessus,
avec l'eau, éventuellement en présence d'une substance auxiliaire de saponification et en la présence éventuelle d'un solvant organique, ou bien
(e) au cas ou dans la formule (I), Z représente le groupement -CO-$Z^1$ dans lequel $Z^1$ est un halogène et $R^1$, $R^2$, $R^3$, $R^4$, X et Z ont les définitions indiquées ci-dessus,
on fait réagir des composés de formule (I), dans laquelle Z représente le groupement -CO-$Z^1$ dans lequel $Z^1$ est un groupe hydroxy et $R^1$, $R^2$, $R^3$, $R^4$, X et Z ont les définitions indiquées ci-dessus, avec un agent d'halogénation, éventuellement en présence d'un catalyseur et en la présence éventuelle d'un diluant, ou bien
(f) au cas ou dans la formule (I), Z représente le groupement -CO-$Z^1$, dans lequel $Z^1$ a la définition indiquée ci-dessus à l'exception d'un halogène et $R^1$, $R^2$, $R^3$, $R^4$, X et Y ont les définitions indiquées ci-dessus,
on fait réagir des composés de formule (I), dans laquelle Z représente le groupement -CO-$Z^1$ où $Z^1$ est un halogène et $R^1$, $R^2$, $R^3$, $R^4$, X et Y ont la définition indiquée ci-dessus,

avec des composés de formule générale (V)

H - $Z^1$ (V)

dans laquelle

$Z^1$ a la définition indiquée ci-dessus à l'exception d'un halogène,

éventuellement en présence d'un accepteur d'acide et en la présence éventuelle d'un diluant, ou bien

(g) au cas ou dans la formule (I), Y est un groupe alcènediyle éventuellement ramifié et/ou éventuellement substitué par un halogène et $R^1$, $R^2$, $R^3$, $R^4$, X et Z ont les définitions indiquées ci-dessus, on fait réagir des composés de formule (I) dans laquelle Y est un groupe alcanediyle portant au moins un substituant halogéno et ayant au moins 2 atomes de carbone et $R^1$, $R^2$, $R^3$, $R^4$, X et Z ont les définitions indiquées ci-dessus, avec des bases, le cas échéant en présence de diluants.

**4.** Compositions herbicides caractérisées par une teneur en au moins un dérivé (hétéro)aryloxynaphtalénique de formule (I) suivant les revendications 1 à 3.

**5.** Procédé pour combattre des mauvaises herbes, caractérisé en ce qu'on fait agir des dérivés (hétéro)aryloxynaphtaléniques de formule ( 1 ) suivant les revendications 1 à 3 sur les mauvaises herbes et/ou sur leur milieu.

**6.** Utilisation de dérivés (hétéro)aryloxynaphtaléniques de formule (I) suivant les revendications 1 à 3 pour combattre des mauvaises herbes.

**7.** Procédé de préparation de compositions herbicides, caractérisé en ce qu'on mélange des dérivés (hétéro)aryloxynaphtaléniques de formule (I) suivant les revendications 1 à 3 avec des diluants et/ou des agents tensio-actifs.

**8.** (Hétéro)aryloxynaphtylamines de formule (II)

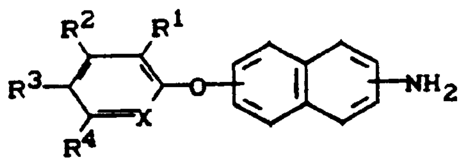

(II)

dans laquelle

$R^1$ est l'hydrogène, un halogène, un groupe cyano ou trifluorométhyle,

$R^2$ est l'hydrogène ou un halogène,

$R^3$ est un halogène, un groupe trifluorométhyle, trifluorométhoxy, trifluorométhylthio ou trifluorométhylsulfonyle,

$R^4$ est l'hydrogène ou un halogène et

X est l'azote ou le groupement C-$R^5$, dans lequel

$R^5$ est l'hydrogène ou un halogène.

**9.** Procédé de production de (hétéro)aryloxynaphtylamines de formule (II)

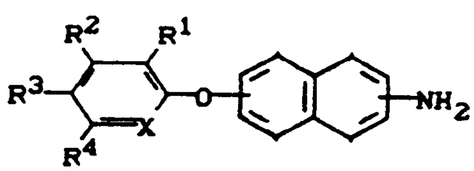

(II)

dans laquelle

$R^1$ est l'hydrogène, un halogène, un groupe cyano ou trifluorométhyle,

$R^2$ est l'hydrogène ou un halogène

$R^3$ est un halogène, un groupe trifluorométhyle, trifluorométhoxy, trifluorométhylthio ou trifluorométhylsulfonyle,

$R^4$ est l'hydrogène ou un halogène et
X est l'azote ou le groupement C-$R^5$, dans lequel
$R^5$ est l'hydrogène ou un halogène,
caractérisé en ce qu'on fait réagir des composés halogéno(hétéro)aryliques de formule (VI)

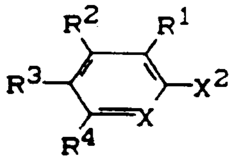

(VI)

dans laquelle
$R^1$, $R^2$, $R^3$, $R^4$ et X ont les définitions indiquées ci-dessus et
$X^2$ est le fluor ou le chlore,
avec des hydroxynaphtylamines de formule générale (VII)

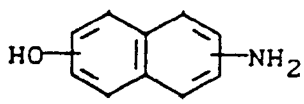

(VII)

ou avec leurs halogénhydrates,
en présence d'un accepteur d'acide et en présence d'un diluant.